# EUROPEAN PATENT APPLICATION

(11) **EP 4 286 513 A1**
(43) Date of publication of application: **06.12.2023**
(21) Application number: 22177191.8
(22) Date of filing: 03.06.2022
(51) Int. Cl.: C12N 5/079, C12N 5/071, C12N 5/0793, C12N 5/074, G01N 33/50

(54) **TRIPLE TISSUE CULTURE FUSION**

(71) Applicant: IMBA-Institut für Molekulare Biotechnologie GmbH, 1030 Wien (AT)
(72) Inventor: KNOBLICH, Jürgen, 2340 Mödling (AT); REUMANN, Daniel, 1030 Wien (AT)
(74) Representative: SONN Patentanwälte GmbH & Co KG

(57) **Abstract**

The invention provides an *in vitro* method of producing a fused tissue culture with at least three different tissues comprising selecting a spatial shape for attaching the at least three different tissues to each other, placing the at least three different tissues into contact in said spatial shape, culturing the at least three different tissues under conditions that maintains tissue fusion of the at least three different tissues. Further provided is a tissue culture comprising a planar or linear fusion of at least three different neural or neuronal tissues, uses thereof and means for its production.

## Description

### Background

Bagley et al. (Nat Methods, 2017, 14(7): 743-751) and WO 2018/197544 A1 describe fused dorsal-ventral cerebral organoids.

Ao et al. (Lab Chip, 2021, 21: 688) describe the fusion of human forebrain and human mid-brain organoids by acoustofluidics within a hexagonal acoustofluidic device that allows rotation and transportation of an organoid. Xiang et al. (Cell Stem Cell 24, 487-497, 2019) describe a fusion organoid of developing thalamus or cortex.

WO 2019/246436 A1 describes a fusion of spinal cord spheroids with skeletal muscle cells. The fused spheroid is then subjected to a second fusion with a cortical spheroid.

Miura et al. (Nature Protocols, 2022, 17: 15) and WO 2021/087145 A1 describe the generation of 3D spheroids resembling specific domains of the nervous system and their fusion to an assembloid.

There remains a need to provided tissue cultures that recapitulate improved in vivo characteristics, in particular at tissue interactions, of tissues in contact with different tissues.

### Summary of the invention

The invention provides an *in vitro* method of producing a fused tissue culture with at least three different tissues comprising the steps of selecting a spatial shape for attaching the at least three different tissues to each other, placing the at least three different tissues into contact in said spatial shape, culturing the at least three different tissues under conditions that maintains tissue fusion of the at least three different tissues; and/or comprising the steps of attaching each of the at least three tissues to at least one other of the at least three tissues with applied force pushing tissues together, culturing the at least three different tissues under conditions that maintains tissue fusion of the at least three different tissues. The force may be applied over a period of at least 4 sec.

Further provided is a tissue culture obtainable by such a method. Provided is a tissue culture comprising a planar or linear fusion of at least three different neural or neuronal tissues.

Further provided is an in vitro method of generating a ventral hindbrain tissue culture, comprising inducing neural or neuronal differentiation in pluripotent cells with a neural induction medium comprising at least one TGF-beta inhibitor and a Wnt activator, and allowing the cells during said induction to form a cell aggregate; culturing the cell aggregate to form a tissue culture.

Further provided is a method of investigating neural formation or neural differentiation, comprising introducing a neural stem cell, neural progenitor cell, neuronal cell, neural cell, glial cell, astrocyte or oligodendrocyte into a tissue culture of the invention and monitoring cell differentiation and/or cell growth.

Further provided is a method of testing or screening a candidate compound for influencing neural cells of a fused tissue culture, comprising contacting cells or a tissue in a method of the invention with the candidate compound or contacting a tissue of the invention with the candidate compound and maintaining said contacted tissue in culture, and observing any change in the neural cell as compared to said tissue without contacting by said candidate compound.

Further provided is a carrier, in particular a mold, for tissue fusion comprising a top surface and a recess in said top surface, wherein said recess is elongated and comprises a deepest depression and at least one slope along the direction of the elongation, the at least one slope descending to the deepest depression, wherein the carrier or mold at least at the deepest depression comprises a low- or non-cell-adhesion surface.

Further provided is a kit comprising a carrier or mold of the invention and neural induction medium.

All embodiments of the invention are described together in the following detailed description and all preferred embodiments relate to all embodiments, aspects, methods, tissue cultures, organoids, materials, like carriers or molds and media, uses and kits alike. E.g. kits and materials or their components can be used in or be suitable for inventive methods. Any component used in the described methods can be part of the kit or describe the materials or their uses. Inventive tissue cultures or organoids are the results of inventive methods or can be used in inventive methods and uses. Preferred and detailed descriptions of the inventive methods read alike on suitability of resulting or used organoids or tissue cultures of the invention. All embodiments can be combined with each other, except where otherwise stated.

### Detailed description

The present invention provides an *in vitro* method of producing a fused tissue culture with at least three different tissues comprising selecting a spatial shape for attaching the at least three different tissues to each other, placing the at least three different tissues into contact in said spatial shape, culturing the at least three different tissues under conditions that maintain tissue fusion of the at least three different tissues. The tissues are cellular tissues. The cells should be alive and functional in order to study their behaviour in the fused tissue culture. The invention allows to select special shapes in which the different tissues can be fused together, in the shape during fusion. This shape will also determine functionality of the produced fused tissue culture as the connectivity between or orientation of the different tissue has an effect of possible functions of the fusion product, i.e. the fused tissue culture. The inventive spatial placement allows the coterminal fusion of the at least three different tissues. In previous methods mentioned in the background section only double fusions were possible at a given time. Such fusions were possible one at a time, and such fusions could be repeated to achieve multiple tissues, but no simultaneous fusions were described in a spatially defined manner.

For example, the inventive spatial placement allows contacts or connections between all three of at least three different tissues (or of more tissues) or more limited contacts, such as with at least one, preferably at least two, of the at least three different tissues being in contact with only one of the (other) at least three different tissues. Such a shape is e.g. a chain of different tissues that are fused to each other. A possible spatial shape is a planar or linear shape.

A linear shape of different tissues or tissue parts of different origin in the produced fused tissue culture is to be understood as a shape wherein a line can be drawn through the arrangement of the different tissues or fused tissue culture wherein the line passes through all or some of the at least three different tissues or tissue parts. At least 3, preferably at least 4, at least 5 at least 6 or more of the at least three different tissues or tissue parts may comprise one line that can be drawn through the arrangement of all the different tissues or tissue parts. In some embodiments all of the different tissues to be fused or tissue parts are found on a shape consisting of 1, 2, 3, 4, 5, 6, 7, 8 or more lines, which lines each can be drawn through the arrangement of the different tissues or tissue parts wherein each line passes through at least 2, preferably at least 3, of the at least three different tissues or tissue parts.

A planar shape of different tissues or tissue parts of different origin in the produced fused tissue culture is to be understood as a shape wherein a plane can be drawn through the arrangement of the different tissues or fused tissue culture wherein the plane passes through all or some of the at least three different tissues or tissue parts. At least 3, preferably at least 4, at least 5 at least 6 or more of the at least three different tissues or tissue parts may comprise one plane that can be drawn through the arrangement of all the different tissues or tissue parts. In some embodiments all of the different tissues to be fused or tissue parts are found on a shape consisting of 1, 2, 3, 4, 5, 6, 7, 8 or more planes, which planes each can be drawn through the arrangement of the different tissues or tissue parts wherein each plane passes through at least 3, preferably at least 4, of the at least three different tissues or tissue parts. The shape may be a surface that is bent as the shape on which the tissues rest on the carrier or recess (bottom surface), or the spatial shape may follow the curvature of a carrier on which the at least three different tissues are placed.

The step of culturing the at least three different tissues under conditions that maintains tissue fusion of the at least three different tissues may comprise standard culturing conditions that allow fusion by growth or attachment through intercellular connections of the different tissues, while maintaining the spatial shape of the at least three different tissues. It may comprise steps to enhance fusion speed, e.g. by using a cellular adhesive, such as extracellular matrix, such as Matrigel, or extracellular matrix components, such as collagen and/or laminin, and/or at least partially removing surface fluid, e.g. by simply removing the medium from the culture, which increases stickiness of the different tissues and contacting the tissues to each other (in the selected spatial shape). The shape during attachment is easily maintained by letting the at least three different tissues rest. Preferably there is no medium stirring or agitation either mechanically or acoustically, that would move the at least three different tissues.

The at least three different tissues may be 3, 4, 5, 6, 7, 8 or more different tissues. The number of different tissues (n) minus 1 (n-1) is the minimum number of fusions that are required to connect all of the different tissues. So, for at least three different tissues, at least n-1 fusions between the different tissues happen. Preferably 2, 3, 4, 5, 6, 7 or more fusions between different tissues happen during the steps of "placing the at least three different tissues into contact in said spatial shape, culturing the at least three different tissues under conditions that maintains tissue fusion of the at least three different tissues". Preferably the different tissue fusions happen simultaneously.

The at least three different tissues may be tissue spheroids or organoids or biopsy tissues or 3D cell aggregates. The different tissues may have a size of 0.1 mm to 10 mm, such as 0.2 mm to 8 mm or 0.3 mm to 6 mm, in their longest dimension. "Size" refers to the longest dimension in 3D space ("length"). Preferably the tissues are globular in shape, in particular with the shortest dimension being not less than 20% of the longest dimension, in particular not less than 30% or not less than 40% of the longest dimension. Other possible shapes are squared or cylindrical, among others. Preferably the volume of the at least three different tissues is at least 1×10⁶ µm³, in particular preferred at least 2×10⁶ µm³, at least 4×10⁶ µm³, at least 6×10⁶ µm³. The fused tissue culture may have the same sizes, shapes and volumes or even larger sizes and volumes, such as a volume of at least 8×10⁶ µm³, at least 10×10⁶ µm³, at least 15×10⁶ µm³ and/or a length of at least 250 µm, especially preferred at least 350 µm.

The at least three different tissues are usually small aggregates of cells and may have a size of at most 12.5 mm, preferably of at most 10000 µm or at most 8000 µm, e.g. with volumes of at most 200 mm³, at most 150 mm³, or at most 125 mm³. In some embodiments, the fused tissue culture may be larger with a size of at most 100 mm, preferably of at most 70 mm or at most 50 mm, e.g. with volumes of at most 6000 mm³, at most 3000 mm³, or at most at most 1000 mm³.

In preferred embodiments, the at least three different tissues are placed onto a carrier, wherein the carrier has a slope gradually descending to a deepest depression, wherein the at least three different tissues sink to the bottom of the carrier and then are pushed together due to a downslope force caused by an inclination of the slope. A carrier with a depression can be used to push the at least three different tissues together by gravity and thereby maintain the spatial shape. The carrier may have a recess or groove that forms the spatial shape in a depression. The recess may be linear, bent, curved or planar, with even more complex forms depending on the number of different tissues to be fused, such as multi-linear of 2, 3 or more lines that intersect, such as star-shaped.

The slope may be lower inclined closer to the deepest depression than further away from the deepest depression of the carrier. At the deepest depression, the inclination may be zero or a horizontal at the bottom of the depression. There may be one or more deepest depressions in the carrier for a given location for the formation of a fused tissue culture. The slope may gradually descend to a deepest depression. Preferably the gradually descending slope is a curved slope. The slope may also be or comprise a linear or planar slope.

The inventive method may comprise placing the at least three different tissues in a recess of a carrier, wherein said recess is elongated and comprises a deepest depression and at least one slope, the at least one slope descending to the deepest depression. Placement of the at least three different tissues into the recess may force contact of each the at least three different tissues with at least one other of the at least three different tissues through gravity in the recess. Through gravity and/or through removal of the residual media in which the at least three tissues are transferred, the at least three different tissues sink to the bottom of the carrier and then are pushed together due to a downslope force caused by an inclination of the slope. The recess helps to put the at least three different tissues into the spatial shape. The recess may have walls, i.e. further inclinations, in sides on which the tissues should not be able to move freely, i.e. to prevent a deviation from the spatial shape. Such walls are e.g. side walls. In particular preferred embodiments, medium can be removed to increase attachment of the different tissue to their respective proximal tissue in contact, as is described further below.

In preferred embodiments the recess has a length of 1 mm to 40 mm, preferably 2 mm to 30 mm, especially preferred 3 mm to 20 mm, and/or a width of 0.5 mm to 16 mm, preferably 1 mm to 12 mm. Such dimensions are usually suitable to accommodate the most common at least three different tissues that are to be fused, such as organoids.

The recess may have a depression (e.g. depth from a top side or top surface of the carrier) of at least 0.2 mm, preferably at least 0.5 mm or even more preferred at least 1 mm, over a length of at least 0.5 mm, preferably at least 2 mm, and/or a depression of at least 0.2 mm, preferably at least 0.5 mm or even more preferred at least 1 mm, over a width of at least 0.3 mm, preferably at least 0.5 mm, especially preferred at least 0.7 mm. Due to the slope, the depression/depth may vary but in these preferred embodiments, over at least the specified length/width (preferably longer/wider) the given dimension is preferably adhered to.

In preferred embodiments, in a cross-sectional view of the carrier the at least one slope comprises a region with a length of at least 2 mm in which region all tangents to the slope enclose an angle between 1° and 20° with a top surface / top side of the carrier. Thus, in a significant part of the recess/the depression, the slope of the depression is at an angle of from 1° to 20°. The top surface / top side is considered as a horizontal. Thus, the angle can also be regarded as 1° and 20° to the horizon. The angle is preferably 1° to 15°, especially preferred 2° to 10°.

The invention further provides an *in vitro* method of producing a fused tissue culture with at least three different tissues comprising attaching each of the at least three tissues to at least one other of the at least three tissues with applied force pushing tissues together, preferably with the force being applied over a period of at least 4 sec, especially preferred at least 5, 6, 7, 8, 9, 10, 12, 14, 16, 18, 20 or more seconds or any range between these values or more, culturing the at least three different tissues under conditions that maintains tissue fusion of the at least three different tissues. This aspect of the invention relates to an alternative to the spatial arrangement described above. Still, it relates to the same principles as the spatial shape aspect and all embodiments described above for the spatial shape aspect also relate to this aspect. Of course, this alternative defining the force can also be combined with the spatial shape mentioned above, e.g. the at least three tissues can be pushed together with the force in the spatial shape. Preferably, the force between two of the at least three different tissues is 0.01 µN to 1000 µN, preferably 0.1 µN to 400 µN, or up to 200 µN. The force may be due to the downslope force acting on the at least three different tissues, e.g. on the carrier/in the recess as described above. The force is preferably applied over a period of at least 10 sec, especially preferred at least 15 sec.

Preferably the carrier or mold (provided as such, or carrier or mold in the kit or uses thereof) has a low- or non-cell-adhesion surface, in particular at the deepest depression or even other regions which may be contacted by cells or tissue cultures, such as the recess. A low- or non-cell-adhesion surface is also referred to as a non-adherent surface. A suitable carrier preferably has a non-adherent surface. A non-adherent surface is a surface on which the cells are placed, and which has little or no adhesion tendency to the cells. Thus, the cells do essentially not attach and/or essentially not adhere to this surface. Without wishing to be bound by theory, use of a non-adherent surface provides a driving force for the cells or tissues to not adhere to the surface, but instead adhere to each other, thus forming or not disturbing a cell aggregate or fused tissue culture in the present invention.

A non-adherent surface may be formed by coating a material with a non-adherent biological or artificial material, or a non-adherent surface may be obtained by suitably shaping a non-adherent material, or by other means known in the art. A carrier or mold on or in which the tissue cultures are maintained or fused will from hereon be called a scaffold. Low-adhesion surfaces are preferably hydrophilic, neutrally charged or non-ionic. They may have a hydrogel layer or coating that repels cell attachment. Such low-adhesion carriers and surfaces are known in the art, e.g. described in WO 2019/014635 or WO 2019/014636.

Scaffolds with a non-adherent surface are made of or are coated with, for example, a silicone, siloxane or polysiloxane, preferably polydimethylsiloxan (PDMS), ethylene oxide, propylene oxide, polyethylene glycol, (PEG)-(co)polymers (for instance PLL-g-(PEG)), poly(ethylene oxide) (PEO) (co)polymers, agarose hydrogels, temperature-responsive materials below their Lower Critical Solution Temperatures (LCST) (for example Poly(N-isopropylacrylamide)), hydrophobic materials (for example olefin polymers), cell-repellent micro- and nanotopographies. A non-adherent surface may comprise a coating with albumin, e.g. bovine albumin, agar, a polyvinyl alcohol (PVA), or a surfactant.

Thus, forming a tissue culture according to the present invention is preferably achieved in a non-adherent scaffold. A non-adherent scaffold has at least one surface that essentially does not allow for the adherence of cells. Preferably, this is the side on or in which cells to form the tissue culture to be formed or fused are placed. A non-adherent scaffold can be formed from a non-adhering material, or can be formed from another material coated with a non-adherent material. A non-adherent carrier, petri dish, tube, or recessed carrier may for example be used as scaffold, but preferably, apart from the groove, the scaffold has a plate-like shape, such as for instance a more or less hexagonal, pentagonal, square, rectangular, triangular, oval or round shape. The carrier/scaffold preferably is made by a material which is bioinert or biocompatible, thus having no or low unwanted interaction with the tissues besides providing a carrier to rest on or holding the medium.

In preferred embodiments of the inventive method a contact point between two of the at least three different tissues is higher than a level of medium fluid. Accordingly, the contact between the at least three different tissues is not immersed in the medium fluid. Of course, medium may be present due to surface tension and capillary effects but most of the medium fluid will descend below the contact point due to gravity. With a nonimmersed contact point, the different tissues have an increased stickiness and a higher propensity to attach to each other, thereby increasing adherence and fusion of the tissues. In practical embodiments, medium fluid may be removed from the tissues and/or from the recess. In other embodiments, the contact point between different tissues is immersed in a medium for fusion.

The present invention is preferably used on brain tissues, such as neural tissues or neuronal tissues. E.g., preferably the at least three different tissues comprise 1, 2 or 3 different brain tissues or neural tissues or neuronal tissues. Other non-neural or non-neuronal tissues may also be fused to at least one brain tissue or neural tissue or neuronal tissue. Such non-neural or non-neuronal tissues are further described below and may e.g. stem from biopsies. They may be from tissues that form connections, in particular axon connections, with the brain tissue or neural tissue or neuronal tissue.

"Neural" is often referred to cells of the brain, which is not limited to neurons, whereas "neuronal" refers to neurons and cells involved with neurons. Astrocytes and glia cells are included in the group of neural cells, but not neuronal cells. Neural stem cells e.g. can give rise to neurons, glial cells (e.g. astrocytes, oligodendrocytes) and other neural stem cells. Neuronal on the other hand is exclusively for neurons. Neuronal stem cell only give rise to neurons, but not glial cells. A neuronal tissue comprises neurons and/or neuronal stem cells. The term neural cells includes neuronal cells. The term neural tissue includes neuronal tissue. Neural differentiation may lead to neural and/or neuronal cells. Neuronal cells are one of the more interesting research subjects of the invention, as they are involved with complex interactions between the different tissues, i.e. the tissue parts of the fused tissue culture that stem from the different tissues originally used in the inventive method. Such interactions are e.g. cell migration from one tissue type to another (as e.g. described in Bagley et al., Nat Methods, 2017, 14(7): 743-751 and WO 2018/197544 A1) and/or axon formation. Axons may reach from one of the different tissues/tissue type to one, two or more of the different tissues/tissue types. "Reaching from" means that the cell bodies are in this tissue/tissue type. The generation of differentiated brain tissues known in the art, e.g. as in the background section and in Xiang et al., Seminars in Cell and Developmental Biology 111 (2021) 40-51; WO 2017/160234 A1. The present invention further provides methods for the generation of differentiated brain tissues. Any differentiated brain tissue, such as organoids or spheroids, generated by a prior art method can be combined with different tissues generated by the present invention.

Preferably, the at least three different tissues are human tissues; likewise, preferably the (fused) tissue culture is a human or non-human primate culture, e.g. a human or non-human primate cell aggregate. The at least three different tissues may stem from culturing human or non-human primate cells, such as pluripotent cells, such as induced pluripotent cells (iPS cells) or embryonic stem cells. Human cells, tissues and cultures are especially preferred.

Gene names or gene symbols as used herein refer to the human genes and are described in databases such as GeneCards (www.genecards.org) or the HGNC database (www.genenames.org). Gene symbols are defined e.g. by the "HUGO Gene Nomenclature Committee" (HGNC). Other designations, such as long names, can be found at their website.

In particular preferred embodiments at least one of the different tissues contains dopaminergic neuronal tissue or a serotonergic neuronal tissue. A dopaminergic neuronal tissue may be for example a ventral midbrain tissue. A dopaminergic neuronal tissue may comprise TH+ cells. A serotonergic neuronal tissue may be for example a ventral hindbrain tissue. A serotonergic neuronal tissue may comprise TPH2 positive cells (Tryptophan Hydroxylase 2 positive cells) or 5-HT positive cells (serotonin positive cells).

The dopaminergic neuronal tissue may comprise dopaminergic cells. The dopaminergic cells in some embodiments are A9 dopaminergic neurons and/or A10 dopaminergic neurons. A9 dopaminergic neurons may be in a tissue resembling functionally and/or morphologically the substantia nigra compacta. The resemblance may be with regard to A9 dopaminergic neuron function and/or cell connectivity. A9 dopaminergic neurons are associated with the nigrostriatal pathway and project into the dorsolateral striatum, where they have a crucial function in fine motor control (Arenas, Denham and Villaescusa, Development 142, 1918-36, 2015). These A9 dopaminergic neurons degenerate in Parkinson's disease (Lees, Shin and Revesz, Lancet 373, 2055-66, 2019). A10 dopaminergic neurons may be in a tissue resembling functionally and/or morphologically the ventral tegmental area. A10 dopaminergic neurons may project to the striatum tissue, especially a ventral striatum, as well as into the cortex tissue, majorly into the limbic system and prefrontal cortex. A10 dopaminergic neurons may be in a tissue resembling functionally and/or morphologically the ventral tegmental area. The resemblance may be with regard to A10 dopaminergic neuron function and/or cell connectivity. A10 dopaminergic neurons are associated with affective encoding ("dopaminergic reward pathway") (Lio, Shin and Ikemoto, Neuropsychopharmacology 33, 2182-94, 2014).

The invention further provides an *in vitro* method of generating a ventral hindbrain tissue culture, comprising inducing neural differentiation in pluripotent cells or multipotent neural stem cells with a neural induction medium comprising at least one TGF-beta inhibitor and a Wnt activator, and allowing the cells during said induction to form a cell aggregate; culturing the cell aggregate to form a tissue culture. The pluripotent or multipotent neural stem cells are preferably human cells, such as induced pluripotent cells (iPS cells). The ventral hindbrain tissue culture may be a ventral hindbrain organoid. The inventive ventral hindbrain tissue culture may be one of the at least three different tissues used in the fusion. The fused tissue culture may comprise parts that stem from the inventive ventral hindbrain tissue culture. The generated ventral hindbrain tissue culture preferably has serotonergic neurons.

The TGF-beta inhibitor may be a TGF-beta signaling pathway inhibitor as it inhibits TGF-beta function. It may be an inhibitor of the TGF-beta superfamily pathways. Preferably one or - more preferred - two, or even more, TGF-beta inhibitors are used. In some preferred embodiments it is comprised of or comprises one or more inhibitors of the TGF-beta receptor or a TGF-beta pathway. In preferred embodiments the at least one TGF-beta (pathway) inhibitor is at least one SMAD inhibitor, preferably DMH1 (dorsomorphin homolog 1)) and/or SB431542 (4-[4-(2H-1,3-Benzodioxol-5-yl)-5-(pyridin-2-yl)-1H-imidazol-2-yl]benzamide). Further preferred TGF-beta inhibitors, that are alternatives or combinable with those mentioned before, are Noggin (a Protein that binds and inactivates BMP proteins, which belong to the TGF-beta superfamily), A 83-01 (a small molecule), LDN 193189 (a small molecule) and/or Dorsomorphine. Further TGF-beta inhibitors are known in the art, e.g. as disclosed at www.medchemexpress.com/Targets/TGF-(beta)%20Receptor.html.

Wnt activators are known in the art and described e.g. in Nusse and Clevers, Cell 169, 2017: 985-999. Nusse and Clevers discuss the Wnt/b-catenin signalling pathway and its manipulation in stem cells. The Wnt activator may be a GSK3 inhibitor. Further WNT activators are disclosed at the website web.stanford.edu/group/nusselab/cgi-bin/wnt/smallmolecules. In preferred embodiments the Wnt activator is a WNT ligand, such as WNT-3a, or CHIR99021 (6-[[2-[[4-(2,4-dichlorophenyl)-5-(5-methyl-1H-im-idazol-2-yl)-2-pyrimidinyl]amino]ethyl]amino]-3-pyridinecarboni-trile). Further preferred Wnt activators are WAY-316606, ABC99, IQ1, QS11, SB-216763. The pluripotent cells are preferably as described above, especially embryonic stem cells (ESCs) or induced pluripotent stem cells (iPSCs), in particular preferred human ESCs or iPSCs.

In preferred embodiments, the pluripotent cells or multipotent cells, that emerge from pluripotent cells, are treated with an activator of sonic hedgehog signaling; preferably a Smoothened agonist, e.g. SAG (3-Chloro-N-[trans-4-(methylamino)cyclo-hexyl]-*N*-[[3-(4-pyridinyl)phenyl]methyl]benzo[*b*]thiophene-2-car-boxamide), retinolic acid and/or purmorphamine. This treatment is preferably before treatment with the TGF-beta inhibitor and/or Wnt activator. The activator of sonic hedgehog signaling is preferably a Smoothened agonist, especially preferred SAG (N-Methyl-N'-(3-pyridinylbenzyl)-N'-(3-chlorobenzo[b]thiophene-2-carbonyl)-1,4-diaminocyclohexane) or purmorphamine (PMA), or a combination thereof.

In further preferred embodiments, the cell aggregate is treated with dissolved extracellular matrix, such as Matrigel, or an extracellular matrix component (or Matrigel component), preferably selected from collagen and/or laminin. Dissolved extracellular matrix means that it is in the liquid state. Even more preferred, the cell aggregate is not embedded in an extracellular matrix in a solid or gel state. The aggregate state of the extracellular matrix component may be controlled by the concentration. In stronger diluted form/at lower concentration, the extracellular matrix component may be in liquid or dissolved state and in lower diluted form/at higher concentration, the extracellular matrix component may form a gel and turn solid. The pluripotent cells are preferably as described above, especially embryonic stem cells (ESCs) and induced pluripotent stem cells (iPSCs), in particular human ESCs and iPSCs. cells, in particular human iPS cells.

In a further preferred embodiment, the cells or cell aggregate is not treated with a gamma-secretase inhibitor such as DAPT. This allows a more cell growth driven/less artificially-forced differentiation.

Also provided is an *in vitro* method of generating a ventral midbrain tissue culture, comprising inducing neural differentiation in pluripotent cells or multipotent neural stem cell with a neural induction medium comprising at least one TGF-beta inhibitor and a Wnt activator and a ROCK inhibitor and allowing the cells during said induction to form a cell aggregate; culturing the cell aggregate to form a tissue culture. The TGF-beta inhibitor may be any one as described above, preferably Noggin and/or SB431542, which work particularly well in this method. The Wnt activator may be any one of the above, preferably CHIR99021. The ROCK inhibitor is preferably Y-27632, which works well in this method. In a preferred embodiment, culturing the cell aggregate comprises treating the cell aggregate with a FGF (fibroblast growth factor), preferably FGF8, and/or an activator of sonic hedgehog signaling. The activator of sonic hedgehog signaling may be SAG, especially preferred SAG at a concentration of 100 nM to 1000 nM, preferably 200 nM to 600 nM. In further preferred embodiments, the cell aggregate is treated with dissolved extracellular matrix, such as Matrigel, or an extracellular matrix component (or Matrigel component), preferably selected from collagen and/or laminin. Dissolved extracellular matrix means that it is in the liquid state. Even more preferred, the cell aggregate is not embedded in an extracellular matrix in a solid or gel state. The aggregate state of the extracellular matrix component may be controlled by the concentration. In stronger diluted form/at lower concentration, the extracellular matrix component may be in liquid or dissolved state and in lower diluted form/at higher concentration, the extracellular matrix component may form a gel and turn solid. The pluripotent cells are preferably as described above, especially embryonic stem cells (ESCs) and induced pluripotent stem cells (iPSCs), in particular human ESCs and iPSCs. In a further preferred embodiment, the cells or cell aggregate is not treated with a gamma-secretase inhibitor such as DAPT. This allows a more cell growth driven/less artificially-forced differentiation.

Further provided is an *in vitro* method of generating a striatal tissue culture, comprising inducing neural differentiation in pluripotent cells or multipotent neural stem cell with a neural induction medium comprising at least an activator of sonic hedgehog signaling and a WNT pathway inhibitor and ROCK inhibitor and allowing the cells during said induction to form a cell aggregate and culturing the cell aggregate to form a tissue culture. The activator of sonic hedgehog signaling is preferably SAG. The WNT pathway inhibitor is preferably IWP2. The ROCK inhibitor is preferably Y-27632, which works well in this method. In preferred embodiments, the cell aggregate is treated with dissolved extracellular matrix, such as Matrigel, or an extracellular matrix component or Matrigel component, preferably selected from collagen and/or laminin. Even more preferred wherein the cell aggregate is not embedded in an extracellular matrix in a solid or gel state. Reference is made to the preceding paragraph on the state of extracellular matrix component. In a further preferred embodiment, the cells or cell aggregate is not treated with a gamma-secretase inhibitor such as DAPT. This allows a more cell growth driven/less-artificially-forced differentiation. The pluripotent cells are preferably as described above, especially embryonic stem cells (ESCs) and induced pluripotent stem cells (iPSCs), in particular human ESCs and iPSCs.

WNT pathway inhibitors are disclosed e.g. in
Nusse and Clevers, Cell 169, 2017: 985-999. Further WNT activators are disclosed at the website web.stan-ford.edu/group/nusselab/cgi-bin/wnt/smallmolecules. The WNT pathway inhibitor may be a Porcupine inhibitor or a tankyrase inhibitor and/or Axin inhibitor. The WNT pathway inhibitor is preferably selected from Wnt-C59, IWR-1, XAV939, IWP-2, IWP-4, DKK1 or a combination thereof. Particular preferred is IWP-2.

Preferably, culturing the cell aggregate comprises treating the cell aggregate with a FGF (fibroblast growth factor), preferably FGF4, FGF8, FGF17, FGF18. Especially preferred is FGF4, especially in the method of generating a ventral hindbrain tissue culture. The FGF treatment may be following the formation of a cell aggregate. Some cells of the aggregate may have differentiated towards a neural fate (such as neuronal precursor cells or even further differentiated to neuronal cells). Said differentiation towards a neural fate may have occurred before FGF treatment starts and/or occurs during FGF treatment.

The cell aggregate may be an embryoid body. An embryoid body is a cell aggregate which has the three different germ layers (Ectoderm, Mesoderm, Endoderm). As it is of interest to increase the ectoderm content and reduce or remove mesoderm and endoderm, the inventive methods (e.g. the usage of neural induction medium from early on, preferably from the beginning) results in structures which are mostly comprised of neuroectoderm and no or very low endoderm/mesoderm.

Extracellular matrix (ECM) may comprise collagens, laminins, entactins, glycosaminoglycans (e.g. hyaluronan), proteoglycans (e.g. neurocans), glycoproteins (e.g. tenascin-R), heparin-sulfated proteo-glycans, fibronectin, vitronectin or any combination thereof. Extracellular matrix may be from the Engelbreth-Holm-Swarm tumor or any component thereof such as laminin, collagen, preferably type 4 collagen, entactin, and optionally further heparan-sulfated proteoglycan or any combination thereof. Such an ECM is Matrigel. Matrigel is known in the art (US 4,829,000) and has been used to model 3D heart tissue previously (WO 01/55297 A2) or neural tissue (WO 2014/090993). Contrary to these previous uses, the present invention does not require a 3D matrix formation by ECM. A dissolved ECM or component thereof may be used as treatment agent or coating for or after fusion. Preferably the matrix comprises laminin, collagen and entactin, preferably in concentrations 30%-85% laminin, 3%-50% collagen and optionally entactin, usually 0.5%-10% entactin. Laminin may require the presence of entactin to form a gel if collagen amounts are insufficient for gel forming. For a dissolved ECM, entactin may be absent or below gel forming concentration. Even more preferred, the ECM comprises in parts by weight about 50%-85% laminin, 5%-40% collagen IV, optionally 1%-10% nidogen, optionally 1%-10% heparan sulfate proteoglycan and 0%-10% entactin. All %-values given for the matrix components are in wt.-%. Entactin is an optional bridging molecule that interacts with laminin and collagen.

The inventive ventral hindbrain tissue culture, or any of the at least three different tissues, which are preferably organoids, can be obtained from culturing pluripotent stem cells. In principle, the cells may also be totipotent, if ethical reasons allow, e.g. for non-human mammalian cells.

A "totipotent" cell can differentiate into any cell type in the body, including the germ line following exposure to stimuli like that normally occurring in development. Accordingly, a totipotent cell may be defined as a cell being capable of growing, i.e. developing, into an entire organism.

The cells used in the methods according to the present invention are preferably not totipotent, but pluripotent or at least multipotent. The multipotent cell may be a multipotent neural stem cell. For a dopaminergic tissue, the multipotent cell may be a midbrain dopaminergic (mDA) neuron progenitor cell. Such midbrain dopaminergic (mDA) neuron progenitor cell may for example be used in the generation of ventral midbrain tissues, like spheroids or organoids, as starting cell in the inventive method.

A "pluripotent" cell is not able of growing into an entire organism, but is capable of giving rise to cell types originating from all three germ layers, i.e., mesoderm, endoderm, and ectoderm, and may be capable of giving rise to all cell types of an organism. Pluripotency can be a feature of the cell per se, e.g. in embryonic stem cells, or it can be induced artificially. E.g. in a preferred embodiment of the invention, the pluripotent stem cell is derived from a somatic, multipotent, unipotent or progenitor cell, wherein pluripotency is induced. Such a cell is referred to as induced pluripotent stem cell herein. The somatic, multipotent, unipotent or progenitor cell can e.g. be used from a patient, which is turned into a pluripotent cell, that is subject to the inventive methods. Such a cell or the resulting tissue culture can be studied for abnormalities, e.g. during tissue culture development according to the inventive methods. A patient may e.g. suffer from a neurological disorder or cerebral tissue deformity. Characteristics of said disorder or deformity can be reproduced in the inventive tissue cultures and investigated.

A "multipotent" cell is capable of giving rise to at least one cell type from each of two or more different organs or tissues of an organism, wherein the said cell types may originate from the same or from different germ layers, but is not capable of giving rise to all cell types of an organism. An example of multipotent cells are multipotent neural stem cell, such as midbrain dopaminergic (mDA) neuron progenitor cells.

In contrast, a "unipotent" cell is capable of differentiating to cells of only one cell lineage.

A "progenitor cell" is a cell that, like a stem cell, has the ability to differentiate into a specific type of cell, with limited options to differentiate, with usually only one target cell. A progenitor cell is usually a unipotent cell, it may also be a multipotent cell.

The inventive tissues and/or cells are preferably mammalian, e.g. human or nun-human primate.

With decreasing differentiation capabilities, stem cells differentiate in the following order: totipotent, pluripotent, multipotent, unipotent. During development of the inventive tissue cultures, stem cells differentiate from pluripotent (also totipotent cells are possible) into multipotent neural stem cells, further into unipotent stem cells and subsequently into non-stem tissue cells. Tissue cells may e.g. be neural or neuronal cells or neuroepithelial cells, such as glial cells.

In preferred embodiments, the inventive tissue cultures (the fused tissue culture and the preceding at least three different tissues) are *in vitro* grown. Since tissues are grown from human or mammalian, preferably primate, pluripotent stem cells, this allows growth of human or primate tissue without obtaining human fetal tissue samples.

In a particular preferred embodiment, the cells of the present invention (including all embodiments related thereto), are human cells.

Preferably, the pluripotent cells during aggregate formation are cultured on a low-cell-adhesion surface, and/or as 3D culture. Low-cell-adhesion surfaces, also referred to as non-adherent surfaces, have been described above and the same applies to this embodiment of the invention.

Culturing as 3D culture means that the cells and/or the forming cell aggregates and tissues are not impeded in their growth by binding to a surface. They may remain free floating in a suspended condition so that expansion in all 3D directions is uniformly possible, or, if settled onto the bottom of a carrier surface, do not attach to that surface, again so that expansion in all 3D directions is uniformly possible. Such culturing is in a low-adhesion culture so that the cells, aggregates or tissues do not attach to culture vessel walls. Low-adhesion surfaces are preferably hydrophilic, neutrally charged or non-ionic. They may have a hydrogel layer or coating that repels cell attachment, forcing cells into a suspended state. Such low-adhesion culture vessels are known in the art, e.g. described in WO 2019/014635 or WO 2019/014636. Preferably the bottom of the culturing carrier is round, especially concave. Alternatively, it may be flat or have a V-bottom shape.

Preferably, the neural induction medium comprises glutamine, non-essential amino acids, heparin, transferrin, insulin, vitamins, salts or any combination thereof. Neural induction medium has been described by Eiraku et al. (Cell Stem Cell (2008) 3: 519-532), US 2011/0091869 A1, WO 2011/055855 A1, Lancaster et al. (Nature (2013) 501: 373-379), and WO 2014/090993 A1. Neural induction medium may comprise DMEM/F12 and/or N2 supplement (Price and Brewer. Protocols for Neural Cell Culture: Third Edition. (2001): 255-64), glutamine, non-essential amino acids, heparin, or any combination thereof. In preferred embodiments, the neural induction medium comprises selenin and/or transferrin, and/or insulin. Neural induction medium preferably lacks growth factors that would differentiate neural tissue to a particular neural fate. Such absent growth factors may be any one of Hedgehog, Wnt, TGF-beta (e.g. Bmp signaling), Notch, retinoids, or FGF, or any combination thereof. In other embodiments, e.g. for region-specific differentiation, specific, dose-controlled and temporally restricted Hedgehog, Wnt, TGF-beta, Notch, retinoids, or FGF species or small molecules activating corresponding (sub)pathways may be present. For example, neural induction medium may comprise inorganic salts: CaCl₂, Fe(NO₃)₃, KCl, MgCl₂, NaCl, NaHCO₃, NaH₂PO₄, ZnSO₄; D-Glucose; HEPES; pyruvate; Amino acids: L-Alanine, L-Arginine, L-Asparagine, L-Cysteine, L-Glutamine, L-Glutamate, Glycine, L-Histidine, L-Isoleucine, L-Leucine, L-Lysine, L-Methionine, L-Phenylalanine, L-Proline, L-Serine, L-Threonine, L-Tryptophan, L-Tyrosine, L-Valine; Vitamins: D-Calcium pantothenate, Choline chloride, Folic acid, i-Inositol, Niacinamide, Pyridoxal, Riboflavin, Thiamine, Vitamin B12; or any combination thereof. The medium preferably comprises 1, 2, 3, 4 or more of the group of inorganic salts, 1, 2, 3, 4 or more of the group of Amino acids, and 1, 2, 3, 4 or more of the group of Vitamins. Furthermore, glucose and/or pyruvate are preferred. Insulin, preferably human insulin, may be present to increase glucose uptake. The medium may comprise phenol red. The medium may comprise heparin. Further components of the medium may be selected from transferrin, especially human transferrin, progesterone, putrescine, selenite, or a combination thereof. Preferably the medium comprises (a) Sodium chloride at a concentration of between 70 and 150 mM;
(b) a neuroactive inorganic salt at a concentration of between 0.000001 and 10 mM, selected from the group consisting of Potassium Chloride, Calcium Chloride, Magnesium Sulfate, Magnesium Chloride, Ferric Nitrate, Zinc sulfate, Cupric sulfate, Ferric sulfate, and combinations thereof; (c) Glycine at a concentration of between 0.0001 and 0.05 mM; (d) L-alanine at a concentration of between 0.0001 and 0.05 mM; and (e) L-serine at a concentration of between 0.001 and 0.03 mM. Any component may be present at a concentration that maintains the survival and neural functionality of a neural cell cultured in the medium (EP 2 986 715 B1).

The invention further provides a tissue culture comprising a planar or linear fusion of at least three different neural or neuronal tissues. Such a tissue culture may be obtained from the inventive method, described above as the fused tissue culture. The at least three different tissues mentioned herein for the inventive method form part of the (fused) tissue culture and contribute as different tissue parts of the (fused) tissue culture). They may be different tissue types, i.e. different kinds of tissues. For the tissue culture of the invention these are preferably neural or neuronal tissues, i.e. the "at least three different neural or neuronal tissues" mentioned above. The tissue culture is a cellular tissue culture and is an aggregate (or aggregation) of cells. It may be an organoid, stemming from fused organoids (as at least three different neural or neuronal tissues). The tissue culture may therefore also be referred to as "fused organoid" herein. The same options, embodiments and preferred elements described for the at least three different tissues as described above also apply to the fused tissue culture and relate to its parts and/or at least three different tissues. The tissue culture may of course be cultured further and achieve larger sizes, as possible for in vitro tissue cultures, in particular organoids. No limit in culturing duration of organoids is known to date, with cultivation times of more than 2 years with still functional tissues.

The inventive tissue culture may be used to study interactions between the different parts (the at least three different neural or neuronal tissues). Preferably, the tissue culture comprises one or more neural cells with a cell body in one of said at least three different neural tissues and an axon to another one of said at least three different neural tissues (axonal outgrowth). The axons may interact with target neurons, such as dendrites or cell bodies (axonal innervation). In preferred embodiments, a tissue comprises neuronal cells that project their axons into at least two of the other neural or neuronal tissues. Axon activity and/or axon formation are particular interesting objects of study of the inventive tissue culture. The inventive tissue may be just formed, in particular by fusion and not yet comprise such axons that reach into different tissues. However, preferably the fused tissue culture may have the cells that are capable to form such axons or are in the process to form such axons or contain cells that give rise to cells that are capable to form axons. Examples include axons of dopaminergic cells or serotonergic cells, which are described further herein. Briefly, such axons may form from specific tissues, such as ventral midbrain tissues (for dopaminergic cells, among others) or ventral hindbrain tissues (for serotonergic cells, among others).

In preferred embodiments, the at least three different neural or neuronal tissues are selected from central nervous system tissue, preferably of a telencephalic tissue, in particular preferred of cortical, striatal, lateral ganglionic eminence (LGE), medial ganglionic eminence (MGE) or caudal ganglionic eminence (CGE) tissue; preferably of a diencephalic tissue, especially preferred thalamic, hypothalamic, epithalamic, subthalamic or optic cup tissue; preferably mesencephalic tissue, especially preferred tectum, tegmentum, ventral tegmental area (VTA), substantia nigra (SN), such as SN pars compacta (SNpc), Nucleus ruber tissue; preferably rhombencephalon tissue, especially preferred cerebellum, medulla, pons, raphe nuclei tissue;
preferably spinal cord tissue; or peripheral nervous system tissue; or any combination thereof. Any of these tissues may be combined in the inventive tissue culture, among others. These tissues may also be used in the inventive method for fusion. These tissues may be in the form of an organoid for fusion in the inventive method.

The at least three different tissues of the inventive method or the at least three different brain, neural or neuronal tissues of the inventive fused tissue culture may be fused with non-neuronal or non-neural tissues, e.g. muscle tissue, heart tissue, intestinal tissue, bone, vascular tissues, liver tissue, spleen tissue, kidney tissue, skin tissue. Tissues fused in the inventive tissue culture can be biopsies. Biopsies can be neural or neuronal (like a biopsy of the brain) or non-neuronal or non-neural. An example is to fuse brain regions associated with motor control with spinal cord and/or muscle. Preferably the non-neuronal or non-neural tissues to be fused may comprise nerveendings that connect with the neuronal or neural tissues in the tissue culture.

In preferred embodiments, the (fused) tissue culture comprises cortical circuit tissue; neural retina tissue; dorsoventral forebrain tissue, preferably lateral ganglionic eminence (LGE) tissue, medial ganglionic eminence (MGE) tissue or caudal ganglionic eminence (CGE) tissue; olfactory tissue, preferably comprising olfactory bulb neurons, amygdala tissue, cortex tissue or hippocampus tissue; limbic system tissue, preferably thalamus, hippocampus, amygdala, hypothalamus; dopaminergic system tissue, preferably ventral tegmental area (VTA), substantia nigra (SN), such as SN pars compacta (SNpc) tissue, nucleus accumbens, olfactory tubercle, prefrontal cortex, cortical, caudate nucleus, putamen, hypothalamus, brainstem, spinal cord tissue; serotonergic system tissue, preferably rostral serotonergic group tissue, such as different tissues of the cortex, striatum, amygdala, substantia nigra, pons, hippocampus, entorhinal cortex, locus coeruleus or caudal serotonergic group tissue, such as trigeminal motor nucleus, dorsal nucleus of vagus nerve, intermediolateral nucleus, medulla, mesencephalon; cerebellar pathway tissue, preferably cortical, thalamic, pons, vestibular nuclei tissue; noradrenergic pathway tissue, preferably cerebellum, spinal cord, hippocampus, basal ganglia, cortex tissue; or any combination thereof. Any of these tissues may be combined in the inventive tissue culture, among others. These tissues may also be used in the inventive method for fusion. These tissues may be in the form of an organoid for fusion in the inventive method. Of course, any of these tissues may be combined in a (fused) tissue culture with the tissues mentioned in the preceding paragraph.

The inventive tissues, or the parts that stem from these tissues in the fused tissue culture, may comprise neurons of such a tissue. E.g. a striatum tissue comprises striatal neurons, a ventral hindbrain tissue comprises ventral hindbrain neurons, a ventral midbrain tissue comprises ventral midbrain tissue neurons, a cortex tissue comprises cortex neurons, etc. for any of the above tissues or tissue parts.

In particular preferred embodiments, the at least one of the neural tissues is selected from ventral hindbrain, ventral midbrain, striatum, cortex. These tissues are particular preferred originating tissues and receiving tissues of dopaminergic or serotonergic axons. The originating tissues have cell bodies of dopaminergic or serotonergic cells that form the axons which protrude into the receiving tissue. The receiving tissues have the axon ends with the nerve ends of these cells that connect to cells in the receiving tissues. Originating tissues may be ventral hindbrain and/or ventral midbrain. Receiving tissues may be striatum and/or cortex. Notably, reciprocal connections or reciprocal projections from receiving tissues into originating tissues can and ideally should also occur, mimicking feedbacksignaling and circuit formation in the brain.

Preferably the tissue culture comprises ventral midbrain tissue in contact with striatum tissue and said striatum tissue being in contact with cortex tissue. "Contact" refers to the tissue body contact, e.g. as controlled by the fusion or attachment during fusion. Axons are not counted as contacts. In fact, axons may reach into all other tissues starting from cells in one of the originating tissues that comprise the cell bodies of the axon forming cells. The ventral midbrain tissue in contact with striatum tissue and said striatum tissue being in contact with cortex tissue can be the product of a linear fusion in the order: ventral midbrain tissue - striatum tissue - cortex tissue. Axons may form from the ventral midbrain tissue (contains the cell bodies) to the striatum tissue and/or cortex tissue. Although the ventral midbrain tissue may not be in contact with the cortex tissue, axons may still form between these tissues since axons can be long and reach through or project beside other tissues (here through/beside the striatum tissue). In preferred embodiments, this tissue culture may be further combined with a ventral hindbrain tissue. The ventral hindbrain tissue may be in contact with the ventral midbrain tissue. Such a tissue culture may be product of a linear fusion of: ventral hindbrain tissue - ventral midbrain tissue - striatum tissue - cortex tissue. Ventral hindbrain tissue may contain cells that form axons that reach to cells of the other tissues selected from ventral midbrain tissue, striatum tissue, and cortex tissue. Such axons may be serotonergic axons.

The tissue culture may have a size of 100 µm to 100 mm, preferably 300 µm to 30 mm, in its longest dimension. The tissue culture may be larger than the different tissues from which it is formed. The tissue culture may be cultured after fusion and enlarge through growth. "Size" refers to the longest dimension in 3D space, as mentioned above. The tissue culture may have an elliptical shape with a longest dimension (length) and a widest dimension perpendicular to the longest dimension (width). The width may be 70 µm to 20 mm.

Preferably the tissue culture comprises a neural cell from one of the at least three different neural or neuronal tissues in at least one of the other at least three different neural or neuronal tissues. The neural cell from one of the at least three different neural or neuronal tissues may have migrated to at least one of the other at least three different neural or neuronal tissues. The provided tissue may have migrating or migrated cells. Such cells may have moved to another tissue type (migration recipient tissue) that is not of the same tissue type. The migrated cells may constitute not more than 5% of the cells of the migration recipient tissue (% of cell numbers).

Preferably the at least three different neural or neuronal tissues or the tissue culture, respectively, comprise a neural tissue of a neural or neuronal tissue type and another neural or neuronal tissue of said neural or neuronal tissue type with its cells being modified by a genetic modification (genetically engineered cells). Tissue types are mentioned as above and are selected e.g. from ventral hindbrain tissue, ventral midbrain tissue, striatum tissue, cortex tissue, olfactory tissue, neural retina tissue, etc. According to this embodiment there may be two or more tissues of these types, one being genetically modified (or: genetically engineered) and another being non-modified or having a different genetic modification. Behaviour, growth and activity (e.g. axon formation and or axon activity, such as neuron signalling) of cells of the genetically modified vs. non-modified or differently modified cells can be studied with such a tissue culture. The tissues of this type (mutated, non-mutated, different mutated) may be in contact with a different tissue, such as a tissue that is a recipient for axons of the tissues of this type (genetically modified, non-modified, differently modified).

Preferably the tissue culture comprises dopaminergic neurons and/or serotonergic neurons. Such neurons are a particularly interesting object for study. Dopaminergic neurons may originate from ventral midbrain tissue. Serotonergic neurons may originate from ventral hindbrain tissue. Preferably the dopaminergic neurons connect between ventral midbrain tissue and striatum tissue and/or connect between ventral midbrain tissue and cortex tissue.

Preferably the tissue culture comprises TH+ (Tyrosine Hydroxylase positive) cells, preferably wherein said TH+ cells have axons in a striatal and/or cortical tissue of the tissue culture. Such cells may be dopaminergic cells. Preferably the tissue culture comprises TPH2 positive cells or 5-HT positive cells, preferably wherein said TPH2 positive cells or 5-HT positive cells have axons in a striatal and/or cortical tissue and/or ventral midbrain tissue of the tissue culture. Such cells may be serotonergic cells.

The invention further provides a method of investigating neuron formation, comprising introducing one or more neural stem cell, neural progenitor cell, neuronal cell, neural cell, glial cell, astrocyte or oligodendrocyte into a tissue culture of the invention and monitoring cell differentiation and/or cell growth. In some embodiments, a mixture or combination of these cells may be introduced, e.g. by injection, into a tissue culture of the invention. The introduction of the one or more cells or combinations thereof and monitoring developments, such as neuron maturation and/or activity can be used to study modified (e.g. genetically) or diseased cells or tissue cultures of diseased tissue cultures that receive the introduction of a healthy cell or a diseased cell or a treated diseased cell. Such a disease may be Parkinson's disease (Hiller et al., npj Regenerative Medicine (2022) 24) or Amyotrophic Lateral Sclerosis (ALS). Such studies may be used to investigate a potential cell therapy (e.g. by introducing a healthy cell to a diseased tissue culture) or to increase understanding of diseased cells or diseased tissue cultures. It is also possible to study healthy cells for cell therapy in a normal environment (healthy tissue culture), to study their properties.

The invention further provides a method of testing or screening a candidate compound for influencing neural cells of a fused tissue culture, comprising contacting cells or a tissue in a method of the invention with the candidate compound or contacting a tissue of the invention with the candidate compound and maintaining said contacted tissue in culture, and observing any change in the neural cell as compared to said tissue without contacting by said candidate compound.

Such a method may comprise treating the cells or fused tissue culture with at least one candidate compound. The effects of this method may be compared to the method without the respective at least one candidate compound as control comparison. Otherwise, the control comparison is performed like-wise as is common for controls in order to evaluate the effect of the at least one candidate compound only. The neural cells can be influenced in various ways e.g. morphologically, genetically, in their expression pattern, or through environmental exposure to e.g. drugs or neural or neuronal circuit activity level (e.g. optogenetic, chemogenetic, electrical stimulation, etc.) or epigenetically. The mentioned observation or any study of the inventive tissue or its cells can detect e.g. changes in differentiation, neural morphological features (such as cell shape, axon shape, dendrite shape, neurite shape, processes shape), changes in neural activity (such as neuronal activity), transcriptional changes, protein expression changes, epigenetic, genetic changes and/or neuron maturation changes. Any such change can be observed in the inventive method.

The invention further provides a mold or carrier for tissue fusion comprising a top surface and a recess in said top surface, wherein said recess is elongated and comprises a deepest depression and at least one slope along the direction of the elongation, the at least one slope descending to the deepest depression, wherein the mold/carrier at least at the deepest depression comprises a low- or non-cell-adhesion surface. Low- or non-cell-adhesion surfaces are described above and the same applies here. E.g. a preferred surface is of or comprises polydimethylsiloxan (PDMS, such as provided in the Sylgard 184 Elastomer Kit). In preferred embodiments, the low- or non-cell-adhesion surface is of a coating with a low- or non-cell-adhesion material. The mold or carrier may have the dimensions and/or the recess as mentioned above. The mold or carrier may have one or more recesses of such dimensions, such as 2, 3, 4, 5, 6, 7, 8, 9, 10 or more recesses.

The invention further provides a kit comprising a mold of the invention and neural induction medium. Both, the mold/carrier and the neural induction medium are described above and the same applies to these components of the kit. The kit may further comprise instruction for use, e.g. to perform any method of the invention.

The following numbered embodiments are preferred according to the invention. Any of the numbered embodiments can of course be combined with embodiments and preferred options as described above, or with the corresponding elements of the example section.
1. An *in vitro* method of producing a fused tissue culture with at least three different tissues comprising
   selecting a spatial shape for attaching the at least three different tissues to each other,
   placing the at least three different tissues into contact in said spatial shape,
   culturing the at least three different tissues under conditions that maintains tissue fusion of the at least three different tissues.
2. The method of 1, wherein at least one, preferably at least two, of the at least three different tissues are in contact with only one of the at least three different tissues.
3. The method of 1 or 2, wherein the spatial shape is a planar or linear shape or the spatial shape follows the curvature of a carrier on which the at least three different tissues are placed.
4. The method of any one of 1 to 3, wherein the at least three different tissues are placed onto a carrier, wherein the carrier has a slope gradually descending to a deepest depression, wherein the at least three different tissues sink to the bottom of the carrier and then are pushed together due to a downslope force caused by an inclination of the slope.
5. The method of 4, with the slope being lower inclined closer to the deepest depression than further away from the deepest depression of the carrier
6. The method of 4 or 5, wherein the slope gradually descending to a deepest depression is a curved slope.
7. The method of any one of 1 to 6, comprising placing the at least three different tissues in a recess of a carrier,
   wherein said recess is elongated and comprises a deepest depression and at least one slope, the at least one slope descending to the deepest depression,
   wherein the placement of the at least three different tissues into the recess forces contacts of each of the at least three different tissues with at least one other of the at least three different tissues through gravity in the recess.
8. The method of 7, wherein the recess has a length of 1 mm to 40 mm and/or a width of 0.5 mm to 16 mm.
9. The method of 7 or 8, wherein the recess has a depression of at least 0.2 mm over a length of at least 0.5 mm and/or a depression of at least 0.2 mm over a width of at least 0.3 mm.
10. The method of any one of 4 to 9, wherein in a cross-sectional view of the carrier the at least one slope comprises a region with a length of at least 2 mm in which region all tangents to the slope enclose an angle between 1° and 20° with a top surface of the carrier.
11. The method of any one of 4 to 10, wherein the carrier at least at the deepest depression has a low- or non-cell-adhesion surface.
12. The method of any one of 1 to 11, wherein a contact point between two of the at least three different tissues is higher than a level of medium fluid.
13. The method of any one of 1 to 12, wherein the at least three different tissues comprise 1, 2 or 3 different brain or neural tissues or neuronal tissues.
14. The method of any one of 1 to 13, wherein at least one of the different tissues contains dopaminergic neural or neuronal tissue or a serotonergic neural or neuronal tissue.
15. The method of any one of 1 to 14, wherein the at least three different tissues are tissue spheroids or organoids or biopsy tissues or 3D cell aggregates; and/or wherein the at least three different tissues are 0.1 mm to 10 mm in size in their longest dimension.
16. An in vitro method of generating a ventral hindbrain tissue culture, comprising inducing neural differentiation in pluripotent cells or multipotent neural stem cells with a neural induction medium comprising at least one TGF-beta inhibitor and a Wnt activator, and allowing the cells during said induction to form a cell aggregate;
   culturing the cell aggregate to form a tissue culture.
17. The method of 16, wherein a) the at least one TGF-beta inhibitor is at least one SMAD inhibitor, preferably DMH1 (dorsomorphin homolog 1)) and/or SB431542 (4-[4-(2H-1,3-Benzodioxol-5-yl)-5-(pyridin-2-yl)-1H-imidazol-2-yl]benzamide); and/or
   b) the Wnt activator is a WNT ligand, such as WNT-3a, or CHIR99021 (6-[[2-[[4-(2,4-dichlorophenyl)-5-(5-methyl-1H-imidazol-2-yl)-2-pyrimidinyl]amino]ethyl]amino]-3-pyridinecarbonitrile).
18. The method of 16 or 17, wherein culturing the cell aggregate comprises treating the cell aggregate with a FGF (fibroblast growth factor), preferably FGF4.
19. An in vitro method of generating a ventral midbrain tissue culture, comprising inducing neural differentiation in pluripotent cells or multipotent neural stem cells with a neural induction medium comprising at least one TGF-beta inhibitor, preferably Noggin and/or SB431542, and a Wnt activator, preferably CHIR99021, and a ROCK inhibitor, preferably Y-27632, and allowing the cells during said induction to form a cell aggregate; culturing the cell aggregate to form a tissue culture, preferably wherein culturing the cell aggregate comprises treating the cell aggregate with a FGF (fibroblast growth factor), preferably FGF8 and/or an activator of sonic hedgehog signaling, preferably SAG, especially preferred SAG at a concentration of 10 nM to 100 µM, preferably 200 nM to 1000 nM; especially preferred wherein the cell aggregate is treated with dissolved extracellular matrix or an extracellular matrix component selected from collagen and/or laminin; even more preferred wherein the cell aggregate is not embedded in a extracellular matrix in a solid or gel state.
20. An in vitro method of generating a striatal tissue culture, comprising inducing neural differentiation in pluripotent cells or multipotent neural stem cells with a neural induction medium comprising at least an activator of sonic hedgehog signaling, preferably SAG, and a WNT pathway inhibitor, preferably IWP2, and ROCK inhibitor, preferably Y-27632,
   and allowing the cells during said induction to form a cell aggregate;
   culturing the cell aggregate to form a tissue culture; especially preferred wherein the cell aggregate is treated with dissolved extracellular matrix or an extracellular matrix component selected from collagen and/or laminin; even more preferred wherein the cell aggregate is not embedded in an extracellular matrix in a solid or gel state; and/or further especially preferred wherein the cells or cell aggregate is not treated with a gamma-secretase inhibitor such as DAPT.
21. The method of any one of 16 to 20, wherein the pluripotent cells or multipotent neural stem cells during aggregate formation are cultured on a low-cell-adhesion surface, and/or as a 3D culture.
22. The method of any one of 16 to 21, wherein the pluripotent cells or multipotent neural stem cells are treated with an activator of sonic hedgehog signaling; preferably SAG (3-Chloro-N-[trans-4-(methylamino)cyclohexyl]-*N*-[[3-(4-pyridinyl)phenyl]methyl]benzo[*b*]thiophene-2-carboxamide), retinolic acid and/or purmorphamine.
23. The method of any one of 16 to 22, wherein the neural induction medium comprises inorganic salts, glutamine, non-essential amino acids, heparin, vitamins, or any combination thereof.
24. A tissue culture comprising a planar or linear fusion of at least three different neural or neuronal tissues.
25. The tissue culture of 24, comprising neural cells with a cell body in one of said at least three different neural or neuronal tissues and an axon to another one of said at least three different neural or neuronal tissues, preferably wherein a tissue comprises neural cells that project their axons into at least two of the other neural or neuronal tissues.
26. The tissue culture of 24 or 25, wherein the at least three different neural or neuronal tissues are selected from central nervous system tissue,
   preferably of a telencephalic tissue, in particular preferred of cortical, striatal, lateral ganglionic eminence (LGE), medial ganglionic eminence (MGE) or caudal ganglionic eminence (CGE) tissue;
   preferably of a diencephalic tissue, especially preferred thalamic, hypothalamic, epithalamic, subthalamic or optic cup tissue;
   preferably mesencephalic tissue, especially preferred tectum, tegmentum, ventral tegmental area (VTA), substantia nigra (SN), such as SN pars compacta (SNpc), Nucleus ruber tissue; preferably rhombencephalon tissue, especially preferred cerebellum, medulla, pons, raphe nuclei tissue;
   preferably spinal cord tissue; or
   peripheral nervous system tissue.
27. The tissue culture of any one of 24 to 26, comprising cortical tissue; dorsoventral forebrain tissue, preferably lateral ganglionic eminence (LGE) tissue, medial ganglionic eminence (MGE) tissue or caudal ganglionic eminence (CGE) tissue; olfactory tissue, preferably comprising olfactory bulb neurons, amygdala tissue, cortex tissue or hippocampus tissue; limbic system tissue, preferably thalamus, hippocampus, amygdala, hypothalamus; dopaminergic system tissue, preferably ventral tegmental area (VTA), substantia nigra (SN), such as SN pars compacta (SNpc) tissue, nucleus accumbens, olfactory tubercle, prefrontal cortex, cortical, caudate nucleus, putamen, hypothalamus, brainstem, spinal cord tissue; serotonergic system tissue, preferably rostral serotonergic group tissue, such as cortex, striatum, amygdala, substantia nigra, pons, hippocampus, entorhinal cortex, locus coeruleus or caudal serotonergic group tissue, such as trigeminal motor nucleus, dorsal nucleus of vagus nerve, intermediolateral nucleus, medulla, mesencephalon;
   cerebellar pathway tissue, preferably cortical, thalamic, pons, vestibular nuclei tissue; noradrenergic pathway tissue, preferably cerebellum, spinal cord, hippocamus, basal ganglia, cortex tissue.
28. The tissue culture of any one of 24 to 27, wherein the at least one of the neural or neuronal tissues is selected from ventral hindbrain, ventral midbrain, striatum, cortex.
29. The tissue culture of any one of 24 to 28 having a size of 100 µm to 100 mm, preferably 300 µm to 30 mm, in its longest dimension.
30. The tissue culture of any one of 24 to 29, comprising ventral midbrain tissue in contact with striatum tissue and said striatum tissue being in contact with cortex tissue.
31. The tissue culture of any one of 24 to 30 comprising a neural cell from one of the at least three different neural or neuronal tissues having migrated to at least one of the other at least three different neural or neuronal tissues.
32. The tissue culture of any one of 24 to 31, wherein the at least three different neural or neuronal tissues comprise a neural or neuronal tissue of a neural or neuronal tissue type and another neural or neuronal or neuronal tissue of said neural or neuronal tissue type with its cells being modified by a genetic mutation.
33. The tissue culture of any one of 24 to 32, containing dopaminergic neurons and/or serotonergic neurons.
34. The tissue culture of 33, wherein the dopaminergic neurons connect between ventral midbrain tissue and striatum tissue and/or connect between ventral midbrain tissue and cortex tissue.
35. The tissue culture of any one of 24 to 34 comprising TH+ cells, preferably wherein said TH+ cells have axons in a striatal and/or cortical tissue of the tissue culture.
36. A method of investigating neuron formation, comprising introducing a neural stem cell, neural progenitor cell, neural cell, glial cell, astrocyte or oligodendrocyte into a tissue culture of any one of 24 to 35 and monitoring cell differentiation and/or cell growth.
37. A method of testing or screening a candidate compound for influencing neural cells of a fused tissue culture, comprising contacting cells or a tissue in a method of any one of 1 to 23 with the candidate compound or contacting a tissue of a culture of any one of 24 to 35 with the candidate compound and maintaining said contacted tissue in culture, and observing any change in the neural cell as compared to said tissue without contacting by said candidate compound.
38. A mold for tissue fusion comprising a top surface and a recess in said top surface, wherein said recess is elongated and comprises a deepest depression and at least one slope along the direction of the elongation, the at least one slope descending to the deepest depression, wherein the mold at least at the deepest depression comprises a low- or non-cell-adhesion surface.
39. The mold of 36 wherein the low- or non-cell-adhesion surface is of a coating with a low- or non-cell-adhesion material.
40. A kit comprising a mold of 38 or 39 and neural induction medium.

Throughout the present disclosure, the articles "a", "an", and "the" are used herein to refer to one or to more than one (i.e., to at least one) of the grammatical object of the article.

As used herein, words of approximation such as, without limitation, "about", "substantial" or "substantially" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by e.g. ±10%.

As used herein, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The "comprising" expressions when used on an element in combination with a numerical range of a certain value of that element means that the element is limited to that range while "comprising" still relates to the optional presence of other elements. E.g. the element with a range may be subject to an implicit proviso excluding the presence of that element in an amount outside of that range. As used herein, the phrase "consisting essentially of" requires the specified integer(s) or steps as well as those that do not materially affect the character or function of the claimed invention. As used herein, the closed term "consisting" is used to indicate the presence of the recited elements only.

The present invention is further illustrated by the following examples, without being limited to these embodiments of the invention.

### Figures

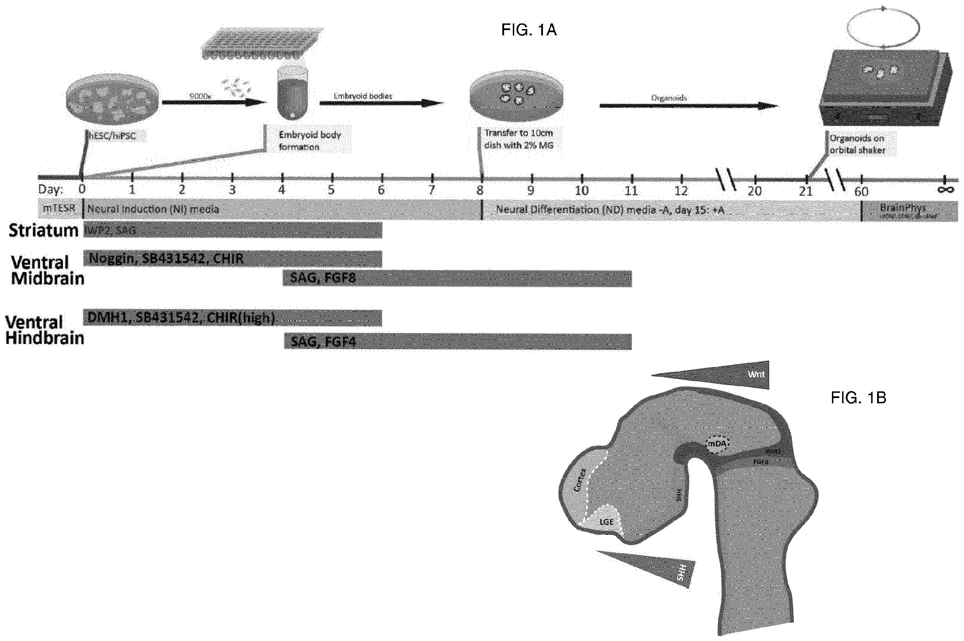

### Figure 1: Patterning schematic

**Figure 1A****:** A patterning schematic for striatal, ventral Midbrain and ventral Hindbrain organoids.

EB formation is performed in media which is permissive or promotes differentiation into ectoderm and neuroectoderm. Such media can be neural induction media (NI).

Both Wnt inhibition and low sonic hedgehog activation at the right time of the protocol were performed to achieve striatal organoids. For ventral midbrain and ventral hindbrain organoids, dual SMAD inhibition is performed by the addition of two TGF-β inhibitors in the first days of the protocol. Such SMAD inhibitors might be Noggin and SB431542. Additionally, Wnt activation is performed. Wnt activation can be performed using the small molecule CHIR99021. After differentiation into neural lineages is started, sonic hedgehog signaling is activated to introduce floor plate, which, together with the right Wnt activation, gives rise to ventral midbrain floor plate progenitors, or at higher posteriorization, hindbrain serotonergic neurons. Sonic hedgehog signaling might be activated by the small molecule SAG (smoothened Agonist). A region-specific FGF such as FGF8 might be applied for increasing the yield of dopaminergic neurons, as has been demonstrated before. A region-specific FGF such as FGF4 might be applied for increasing the yield of serotonergic neurons, as has been demonstrated before.

**Figure 1B****:** Overview of the position of the regions relevant to the dopaminergic system, namely the Cortex, LGE (Lateral Ganglionic Eminence, which gives rise to striatal projection neurons) and ventral Midbrain, giving rise to mDA (midbrain dopaminergic neurons). Figure 1B also provides an overview of the patterning axes and molecules used in the generation of striatal and ventral midbrain organoids.

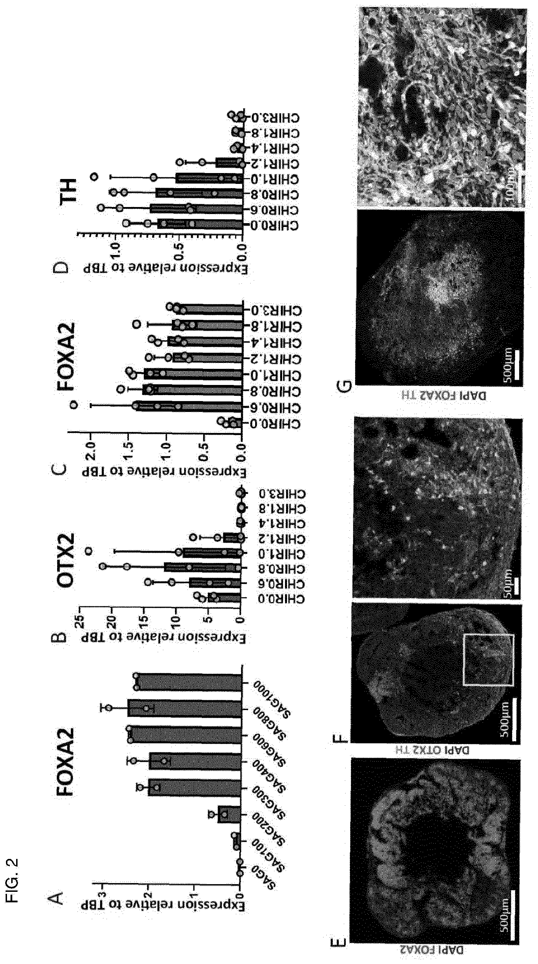

### Figure 2: Generation of ventral midbrain organoids

Figure 2A-D: qPCR analysis of markers of ventral midbrain of the dose curving experiment to generate ventral midbrain organoids. Two separate dose curving experiments were performed, where first the correct dosage of sonic hedgehog activation was tested (Figure 1A, floor plate marker FOXA2). Secondly, we performed qPCR to test for appropriate concentrations of posteriorization using Wnt activation. Dopaminergic neurons are born in the very ventral floor plate of the posterior midbrain, next to the midbrain-hindbrain boundary, thus the Forebrain and Midbrain marker OTX2 should still be expressed, whereas the hindbrain is OTX2 negative. We found a concentration of 0.8-1 µM sufficient to introduce midbrain (Figure 2B), while the floor plate marker FOXA2 was still expressed (Figure 2C). qPCR for the dopaminergic marker TH (Tyrosine hydroxylase, the rate-limiting enzyme for dopamine synthesis) confirmed this concentration of Wnt activation, with a sharp drop of TH expression starting at 1.2 µM CHIR (Figure 2D).

All qPCR data are relative to the expression of TBP. Figure 2E-G: Additional stainings by Immunohistochemistry (IHC) confirmed the expression of FOXA2 and OTX2 in 20-day old organoids. Already on day 20, the first expression of TH (Tyrosine Hydroxylase, the rate-limiting step for dopamine synthesis and a marker for dopaminergic neurons) can be noticed, and TH positive cells can be broadly found on day 40.

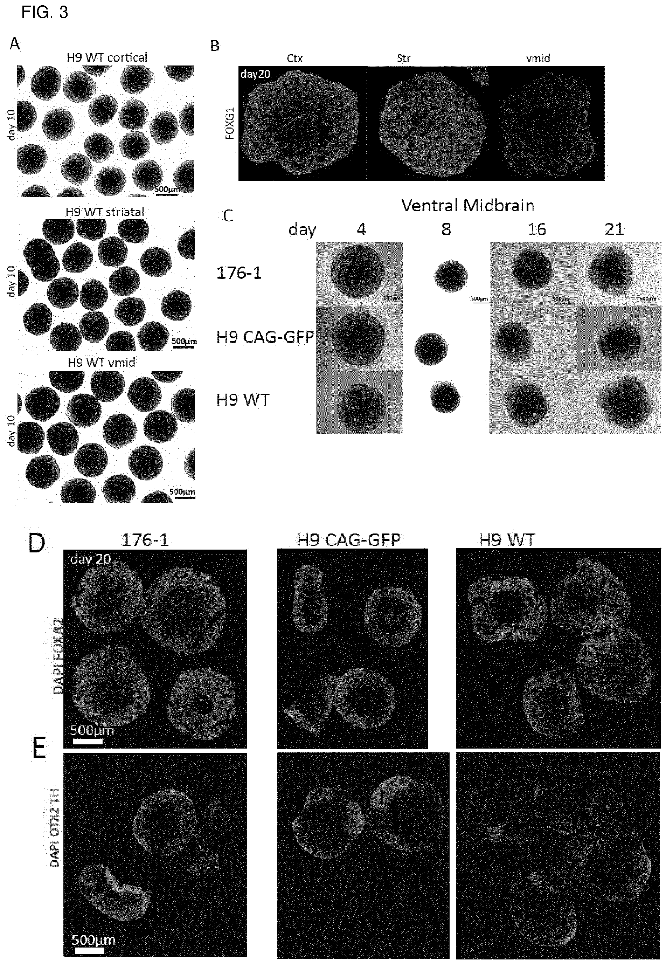

### Figure 3: Further ventral midbrain data

Panel A: Organoid formation of Cortical, Striatal and ventral Midbrain organoids at day 10. All protocols result in homogeneously sized organoids in H9 embryonic stem cells.

Panel B: While striatal and cortical organoids express the forebrain marker FOXG1, correctly patterned ventral midbrain organoids do not express FOXG1.

Panel C: The ventral Midbrain protocol has been tested with one iPSC and two H9 embryonic stem cell derived cell lines.

Panel D: FOXA2 staining of day 20 old ventral midbrain patterned organoids of multiple cell lines shows robust generation of floor plate patterned tissue.

Panel E: Staining for OTX2 and TH indicates robust production of TH⁺ dopaminergic neurons from multiple cell lines.

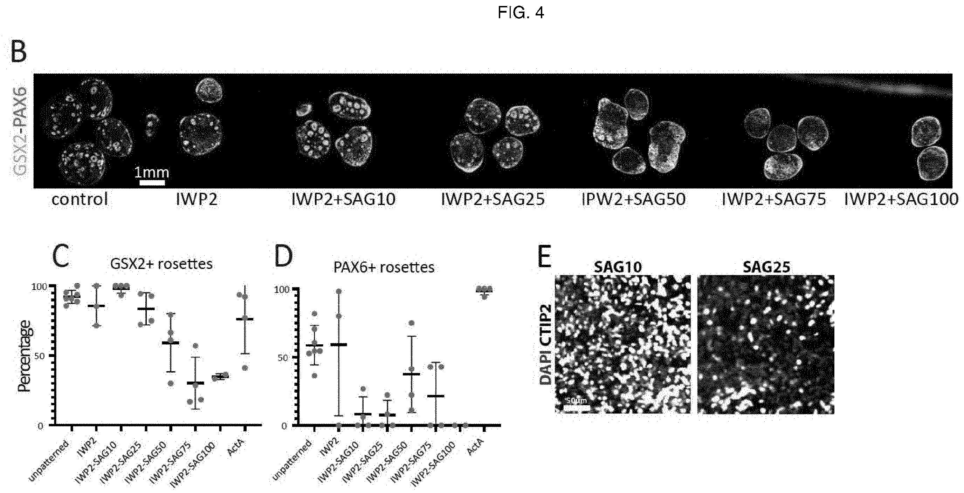

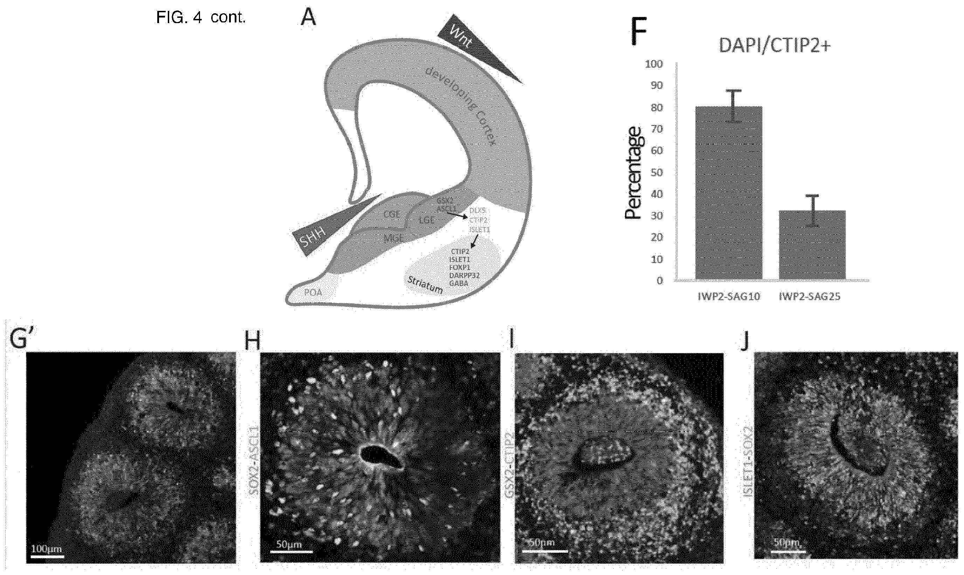

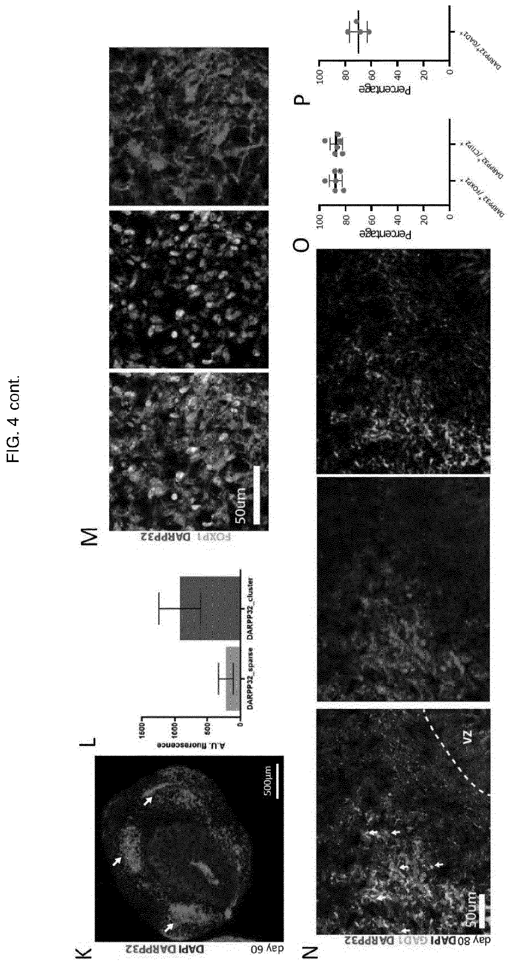

### Figure 4: Generation of striatal organoids

Panel A: Schematic of dorsoventral patterning gradients important for striatal neurogenesis (the caudalizing and dorsalizing factor Wnt, and the ventralizing factor SHH) and markers expressed during neurogenesis of striatal neurons from the LGE (lateral ganglionic eminence) to young bourn neurons to mature striatal neurons.

Panel B: Wnt inhibition together with a dose curve of sonic hedgehog activation with SAG demonstrates that low concentrations of SHH activation were sufficient to induce GSX2⁺ PAX6⁻ LGE neural stem cells in 28 day old organoids.

Panel C, D: Quantifications of GSX2⁺ rosettes and PAX6⁻ rosettes, indicating LGE identity.

Panel E-F: Immunolabeling of CTIP2, an important early striatal marker, in two conditions with promising numbers of GSX2⁺ rosettes, but low PAX6 expression. Notably, CTIP2 expression in 25 nM SAG treated organoids is much lower than in SAG10 treated organoids (Panel F).

Panel G-J: Further marker characterization of day 28 striatal organoids with 10 nM SAG. IWP2-SAG10 treated organoids displayed GSX2⁺ rosettes which produce DLX5⁺ young bourn LGE-derived neurons (Panel G, G'). Further LGE or young striatal neuron markers such as ASCL1 (Panel H), CTIP2 (Panel I) and ISLET (Panel J) are readily expressed in SOX2⁺ neural rosettes.

Panel K: Striatal neurons express DARPP32, a marker for dopamine-sensitive neurons. DARPP32 can be broadly found in day 60 old LGE-patterned organoids and form DARPP32⁺ clusters (white arrows) .

Panel L: We could surprisingly find different expression levels of cells in DARPP32 clusters versus DARPP32⁺ cells which are scattered through the organoid, which could indicate immature and mature striatal neurons.

Panel M: Staining for FOXP1, another important striatal marker, together with DARPP32.

Panel N: Staining for GAD1 and DARPP32 in 80-day old striatal organoids. Striatal neurons are majorly GABAergic, and DARPP32⁺ neurons indeed expressed the marker GAD1 (rate-limiting enzyme for GABA synthesis).

Panel O: Quantification of DARPP32⁺ neurons being positive for FOXP1 and CTIP2.

Panel P: Quantification of DARPP32⁺ neurons being GABAergic (GAD1⁺).

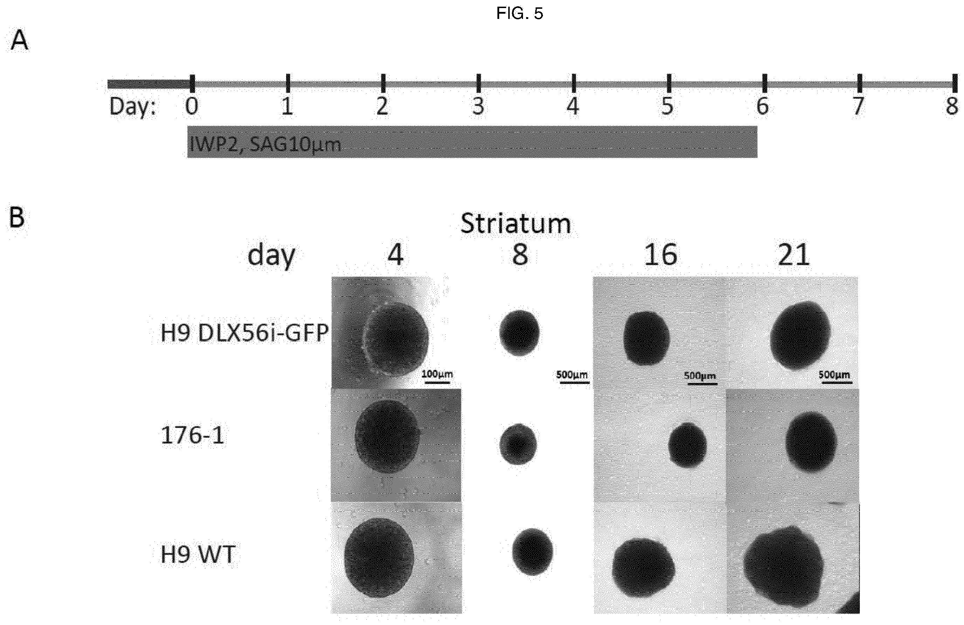

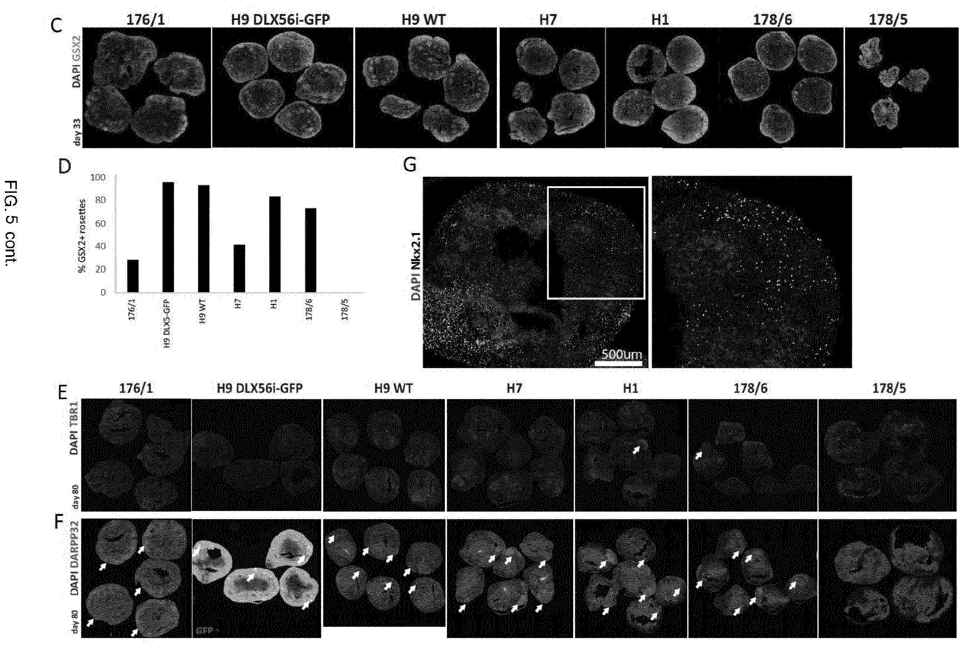

### Figure 5: Further striatum data

Panel A: patterning protocol for the generation of striatal organoids

Panel B: The striatal protocol has been tested with multiple cell lines, example EBs of two H9 derived cell lines and 176-1. Panel C: GSX2 staining of day 33 striatal patterned organoids of 7 cell lines (5 iPSC lines, 2 H9 derived embryonic stem cell lines).

Panel D: 6 out of 7 cell lines displayed GSX2⁺ rosettes on day 33, although with variation in the total percentage of GSX2⁺ rosettes.

Panel E: Striatal patterned organoids do not express the cortical marker TBR1 at day 80, with few exceptions (white arrows). This is of particular importance, as many striatal markers (DARPP32, CTIP2) are also expressed in cortical neurons.

Panel F: Striatal patterned organoids express the striatal marker DARPP32 at day 80 (white arrows).

Panel G: The MGE and interneuron marker Nkx2.1 is expressed scattered throughout striatal organoids at day 60, indicating some tissue in striatal organoids might be of MGE origin.

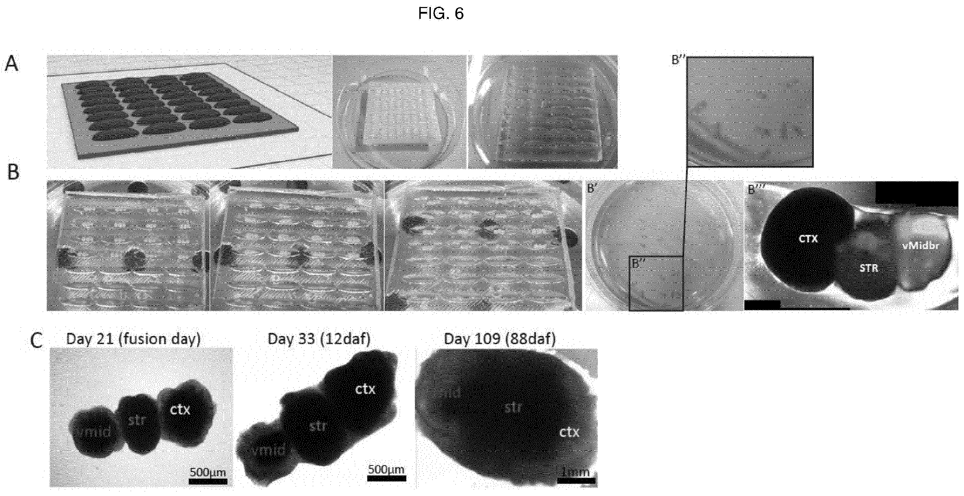

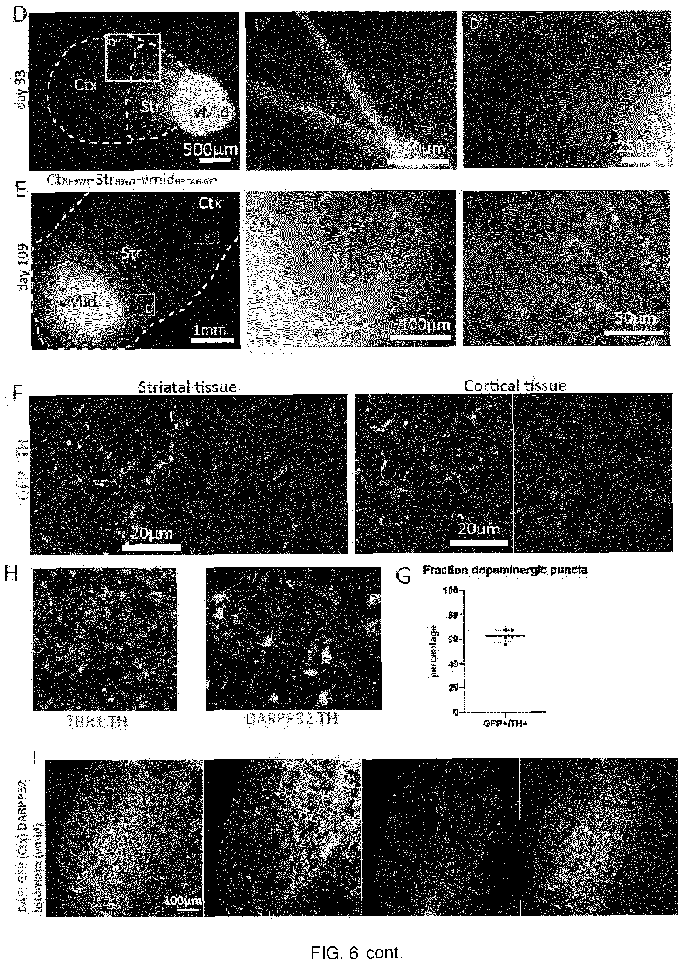

### Figure 6: Linear organoid fusions

Panel A: Generation of fusion molds for fusions of 3 or more organoid regions in a spatially defined manner. Embedding molds were designed using Tinkercad. First, a negative is designed and then sliced using XYZprint^{®} to prepare for 3D printing. The negative was printed using the XYZprinting printer da Vinci Color in full filling mode and high resolution.

Subsequently, the negative was surface treated with a heat gun to melt the surface and create a smooth finish. The positive was then cast using the silicone PDMS (Polydimethylsiloxane).

Prior to usage, the positive mold was washed with 70% Ethanol and coated with an anti-adherence solution to further increase the nonstick characteristics of PDMS.

Panel B: For spatially defined/ linear fusions, organoids were linearly put into the molds. Media is then removed and the organoids were attached for 30sec - lmin. Subsequently, a small volume of Matrigel is added to the organoids. After solidification of the Matrigel at 37°C, the organoids can be washed out carefully with Improved+A media. For the first 2 days, organoids were cultured without shaking, after 2 days the organoids can be transferred back on an orbital shaker. Panel B': a plate with triple fusions after transfer. B": magnified view on linearly fused organoids. B‴: a linear fusion with Cortex(unlabeled):: Striatum(CAG-tdtomato)::ventral Midbrain(CAG-GFP). Fluorophore channels were added to the red or green channel respectively. Panel C: Triple fusions on the day of fusion, 12 days after fusion and 88 days after fusion. The three different tissues start to grow together and form a homogeneous tissue.

Panel D: Triple fusions where the ventral midbrain is labelled with GFP (H9 CAG-GFP). Only 12 days after fusion, first axon bundles emerge and project into striatal and cortical tissue. Panel E: Triple fusions on day 109, the striatal and cortical tissue is highly innervated.

Panel F: GFP labeled projections from the ventral midbrain broadly express TH in striatal and cortical tissues (quantifications in Panel G).

Panel H: TH⁺ projections highly innervate TBR1⁺ cortical regions and DARPP32⁺ striatal regions.

I: In Ctx_{CAG-GFP}::Striatum::vMid_{CAG-tdtomato} fusions, the striatum is highly innervated in triple fusions by cortical and ventral midbrain, indicating the formation of complex neuronal circuits.

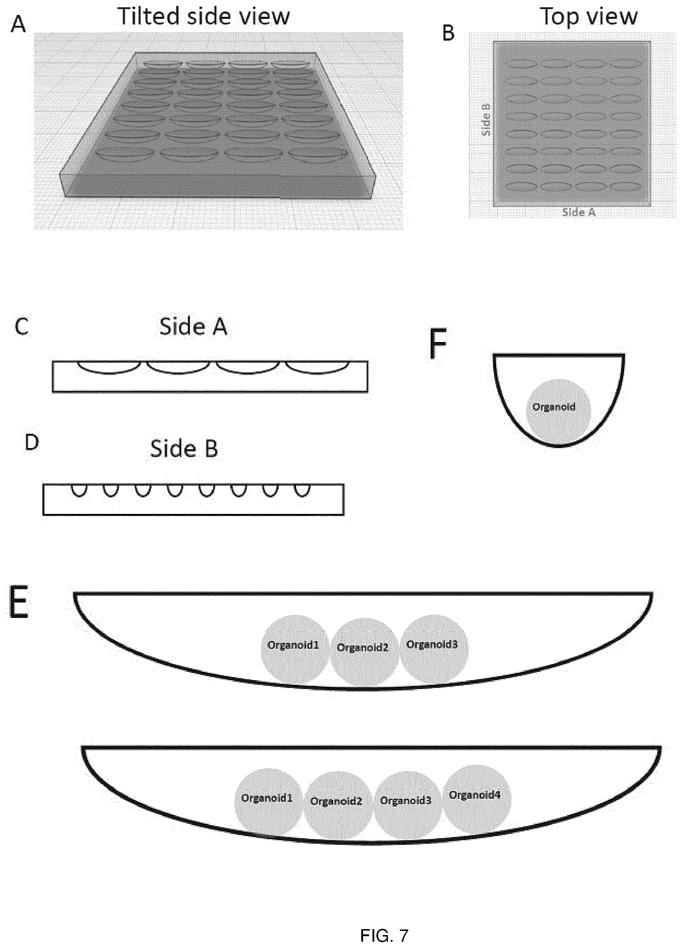

### Figure 7: Embedding mold design

Panel A: Tilted side view of the PDMS embedding mold.

Panel B: Top view of the PDMS embedding mold.

Panel C: Side view (side A).

Panel D: Side view (Side B).

Panel E: Design of individual molds. Focus has been put on a low curvature at the side where organoids are positioned, allowing organoids to be fused in a straight line, but tilted sides, which push organoids together. Examples of triple and quadruple fusions are shown.

Panel F: Side view.

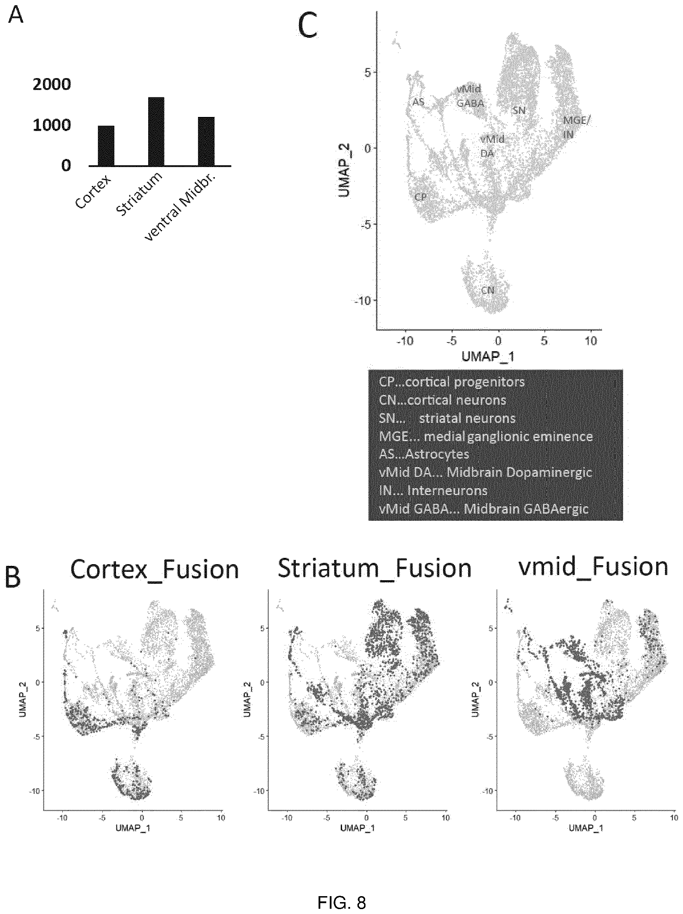

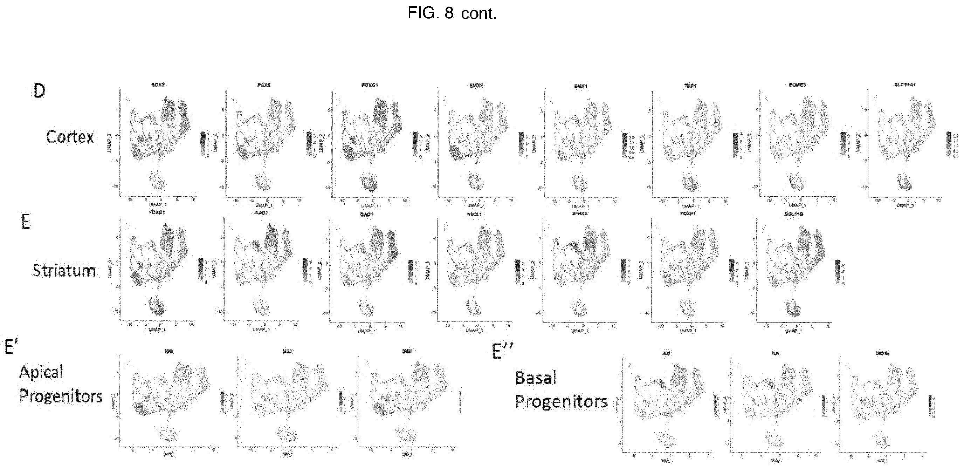

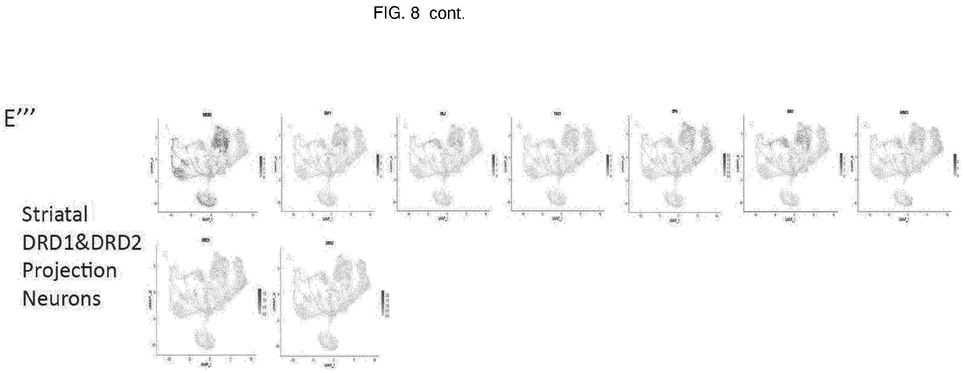

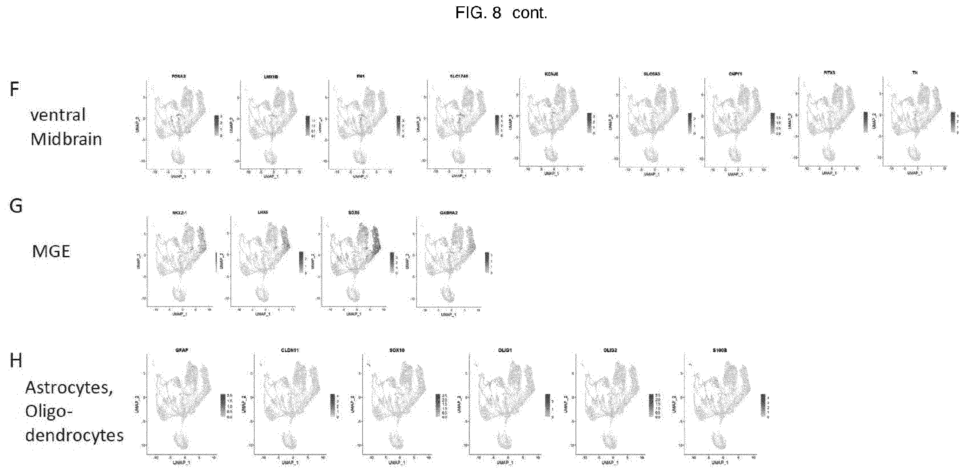

### Figure 8: scRNAseq

Panel A: For scRNAseq, linear fusions were cut apart and the individual regions were barcoded using Multiseq. After filtering, about 1000 cells per region were recovered.

Panel B: Representation of cortical, striatal and ventral midbrain tissue of triple fusions in a UMAP.

Panel C: identities of cell clusters found in the experiment (for marker expression, see panel D-I). We found clusters of cortical progenitors (CP), cortical neurons (CN), striatal neurons (SN), medial ganglionic eminence (MGE) derived interneurons (IN), Astrocytes (AS) and midbrain dopaminergic neurons (mDA).

Panel D: Overview of expression of neural progenitors (SOX2), forebrain (FOXG1), dorsal forebrain (FOXG1+PAX6) and the cortical progenitor and neuron marker EMX1 and EMX2, EOMES, TBR1 and SLC17A7 (VGLUT1).

Panel E: Overview expression of main striatal linage markers. Striatal progenitors express FOXG1 and become GABAergic (GAD1, GAD2) neurons which express ISL1, EBF1, ASCL1, ZFHX3, FOXP1 and BCL11B (CTIP2). Striatal neurons are born by apical progenitors (E') and basal progenitors (E"), which produce interneurons and produce two different types of striatal medium spiny neurons (MSNs, Panel E‴): Dopamine receptor type 1 MSNs (EBF1, ISL1, TAC1) and Dopamine receptor type 2 striatal projection neurons (SP9, SIX3, GIRK3). While 60 days is still young, first neurons could be observed expressing both of these receptors (DRD1, DRD2).

Panel F: The ventral midbrain clusters expressed the floor plate marker FOXA2 and the dopaminergic progenitor marker LMX1B and EN1 as well as SLC17A8, KCNJ6, SLC6A3 as well as CNPY1, PITX3 and TH.

Panel G: The MGE cluster expresses MGE specific genes such as NKX2.1, LHX6, SOX6 and SOX2.

Panel H: We could additionally find a small cluster of astrocyte and oligodendrocyte precursors, expressing GFAP, CLDN11, SOX10, OLIG1, OLIG2 and S100B.

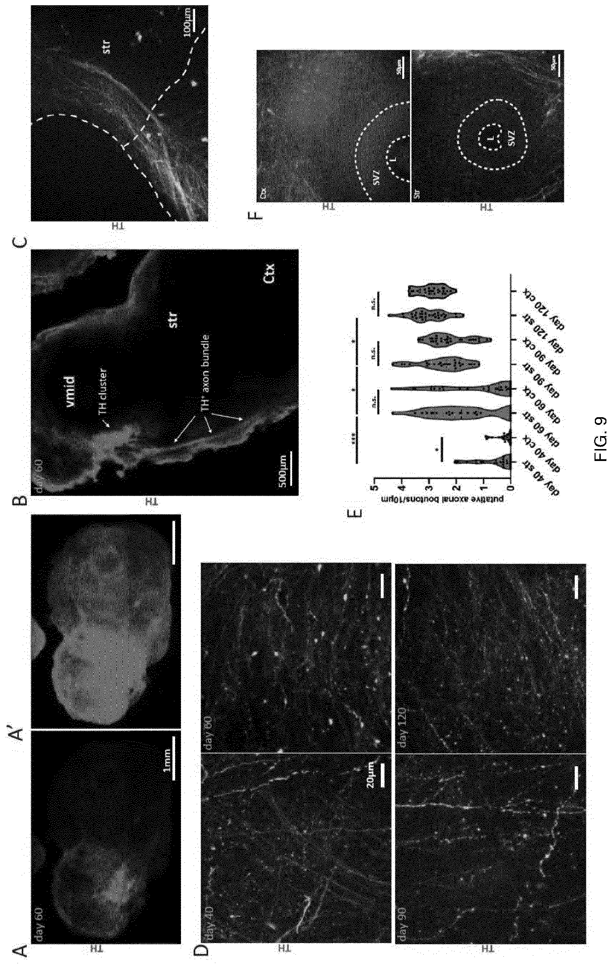

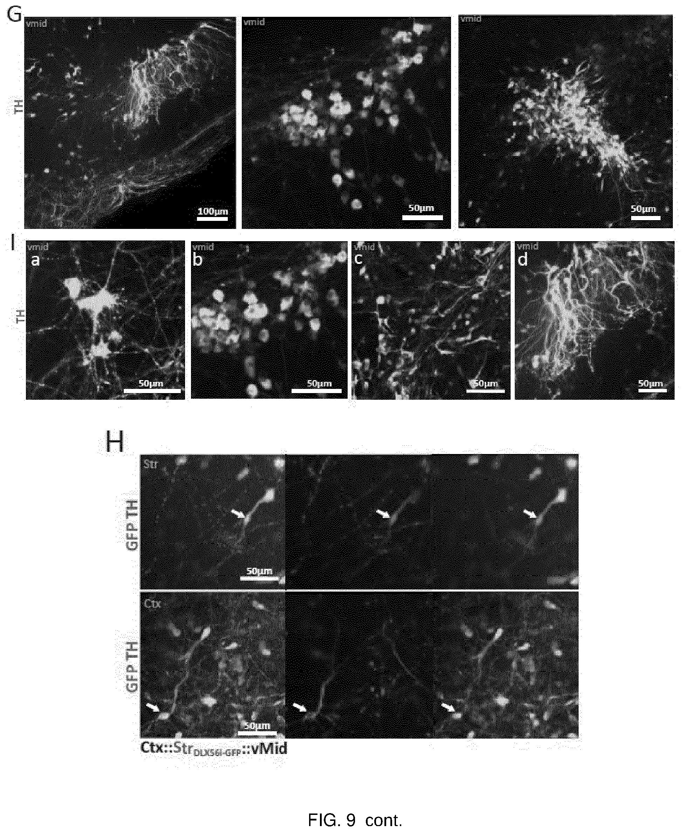

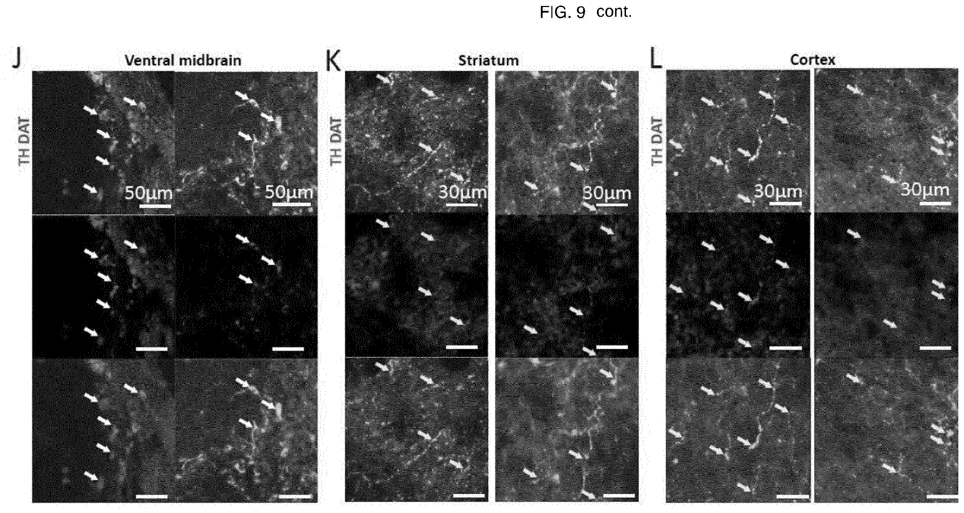

### Figure 9: Tissue clearing and morphological reconstruction

Panel A: Whole-mount 2ECi tissue clearing and z projection of a triple fusion with immunostaining for TH. Left side: low intensity demonstrates TH⁺ clusters in the ventral midbrain of the fusion. Right side: High intensity demonstrates innervation of striatal and cortical tissue.

Panel B: Whole-mount 2ECi tissue clearing reveals TH⁺ axon bundles projecting from TH clusters into striatal and cortical tissues.

Panel C: high magnification recording of a TH⁺ axon bundle originating from the ventral midbrain side and projecting to the striatum.

Panel D: 2ECi tissue clearing with immunolabeling for TH⁺ axons in the striatum from day 40 to day 120. Over time, more putative axonal boutons appear on TH⁺ axons.

Panel E: Quantification of putative axonal boutons per 10µm of axon length. Over time, the amount of putative axonal boutons significantly increases in both striatal and cortical tissues.

Panel F: TH⁺ axons not only innervate neuronal tissues but avoid neural progenitor regions such as the subventricular zone (SVZ). L=Lumen.

Panel G: Dopaminergic neurons often, but not always, organize in clusters which can display a multitude of different morphologies.

Panel H: TH⁺ neurons can also be found in the striatum and cortex, however they can be morphologically distinguished from ventral midbrain dopaminergic neurons. Multiple reports of TH positive interneurons in the human cortex exist, thus we grew fusions using a DLX5-GFP cell line, which labels forebrain interneurons. We could confirm that TH⁺ cells in striatum and cortex were GFP⁺ and thus TH⁺ interneurons. TH⁺ interneurons were additionally observed in scRNAseq (see Figure 8F).

Panel I: various morphologies of dopaminergic neurons could be observed such as multipolar (images a, b) and bipolar (c, d) with variations in cellular morphology and cell body size.

Panel J-L: 2D Immunostaining for TH and DAT (Dopamine transporter) in ventral midbrain (J), Striatum (K) and Cortex (L). Double positive cells (J) are highlighted with a white arrow, dopaminergic axons (K and L) which express DAT are highlighted with yellow arrows. DAT is vital for dopamine transport and indicates that dopaminergic neurons matured to a functional level, releasing and re-uptaking dopamine from its synapses.

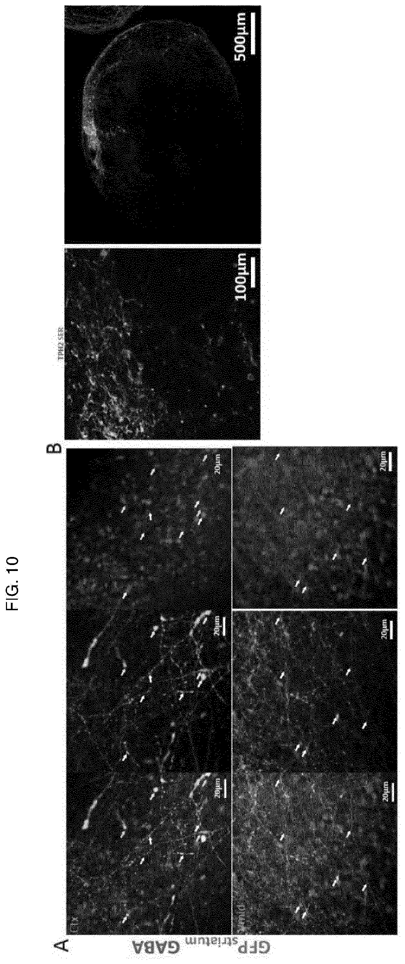

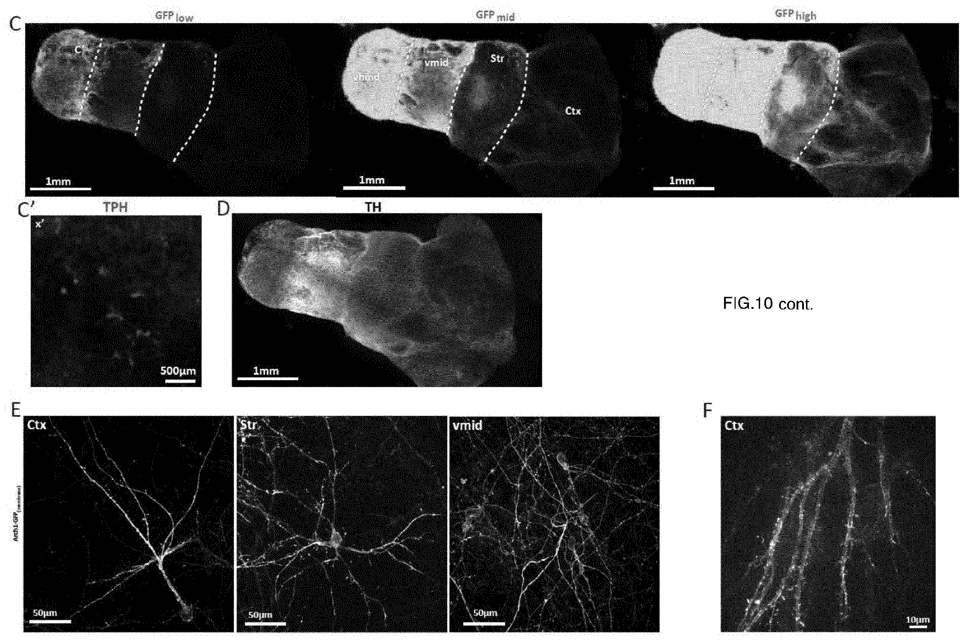

### Figure 10: tissue clearing and morphological reconstruction

Panel A: A triple fusion where the striatum is labeled with CAG-GFP. GFP⁺ projections in 2ECi cleared organoids partially were positive for GABA (white arrows), indicating striatal GABAergic long-range connections. Notably, cortical regions often had GFP GABA double positive cells (yellow arrows) and axons originating from these cells, which are absent in ventral midbrain tissues. Panel B: 2D immunohistochemistry characterization of a representative ventral hindbrain organoid with serotonergic neurons. Serotonergic neurons express TPH2 and produce Serotonin (5-HT). Both markers are broadly expressed in ventral hindbrain organoids.

Panel C: 2ECi cleared quadruple fusion of Ctx::Str::ventral midbrain::ventral hindbrain, with the ventral hindbrain being labeled constitutively with CAG-GFP. Three different intensity levels are displayed, to highlight various features of innervation. Notably, ventral midbrain received substantially more innervation from the ventral hindbrain than striatum and cortex, but all three target regions were densely innervated.

Panel C': Immunolabeling of TPH displays serotonergic neurons in the ventral hindbrain part of the quadruple fusion.

Panel D: Same organoid as in Panel C, immunostaining for TH. Notably, the ventral midbrain shows the highest signal of TH, with innervation of the ventral hindbrain, striatum and cortex.

Panel E: Live imaging of 150-day old organoids infected with a Synapsin-Arch1-GFP AAV. The fusion protein is membrane-bound and allows reconstruction of neuronal morphologies. We could observe various morphologies in the different regions of the fusion. Panel F: High magnification live imaging of a dendritic tree of a cortical neuron with readily observable dendritic spines.

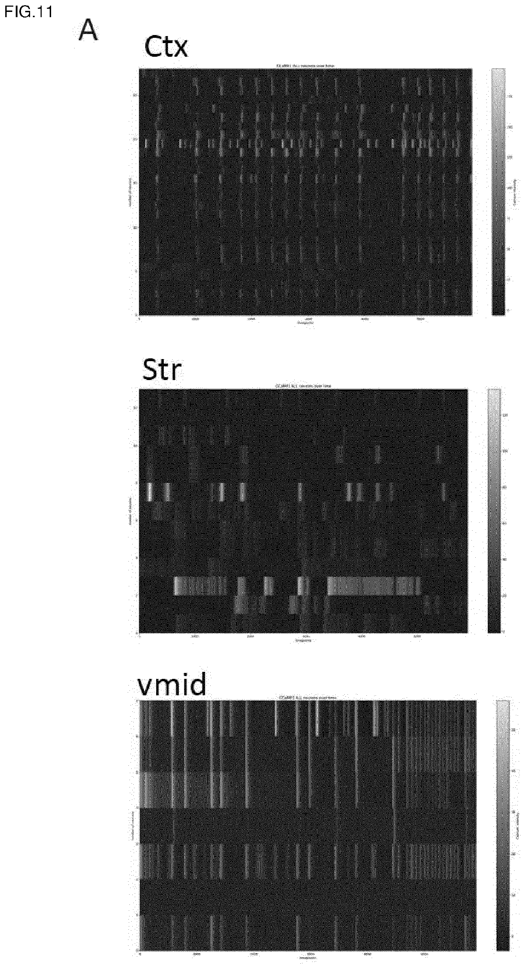

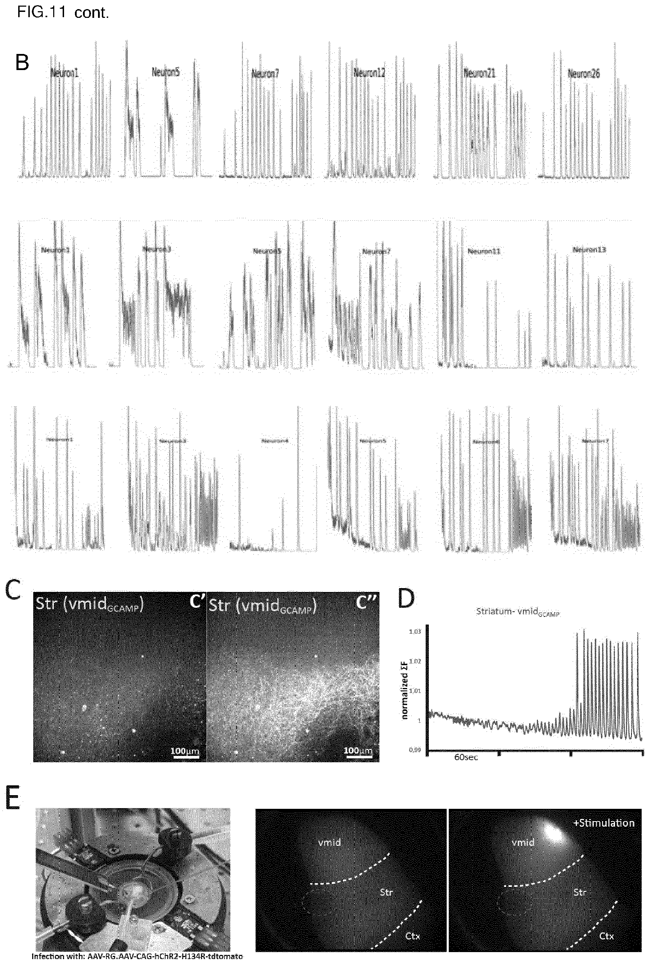

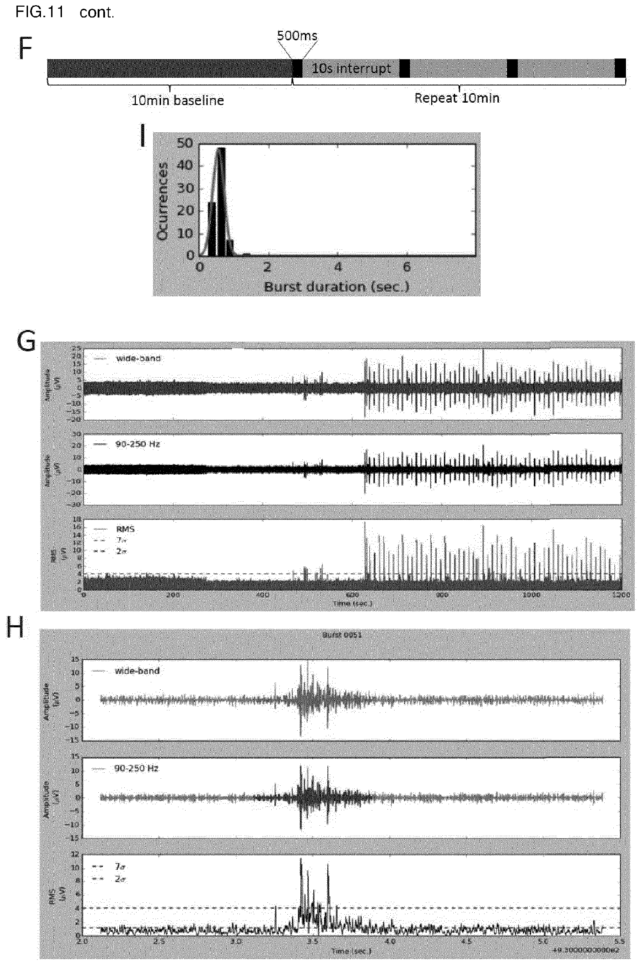

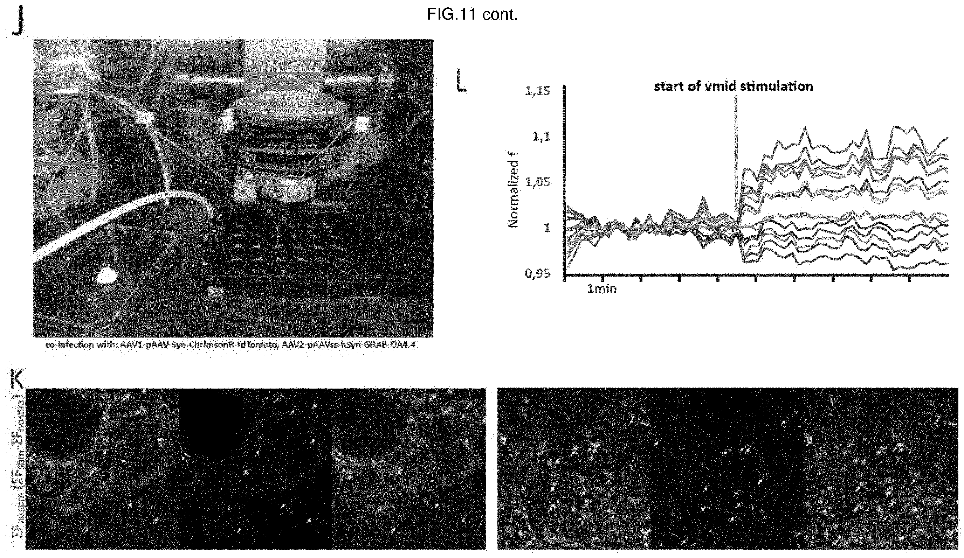

### Figure 11: neural activity

Panel A: Organoids were grown with an H9 Syn-GCAMP cell line in either the cortical, striatal or ventral midbrain region. Recordings were performed for 6.5 min continuously with a frame every 65 ms. Different modalities in activity could be observed in all three regions, with the cortex often displaying synchronous network events, whereas the striatum displayed asynchronous activity, but with often longer calcium events than the other two regions. Ventral midbrain recordings displayed events of highly synchronous activity, lasting for seconds to more than a minute- in this example at the end of the recording.

Panel B: Example traces from A indicating different activity modalities in all three regions.

Panel C: A recording in a striatal organoid with a
Ctx_{WT}::Str_{WT}::ventral Midbrain_{GCAMP} fusion. Network events from axons from the ventral midbrain could be observed (fluorescence change in C' versus C"). No GCAMP⁺ cell bodies were visible in the area of recording.

Panel D: Cumulative fluorescence over time from the recording of Panel C. Notably, no detectable synchronous activity occurs in the first minute of the recording, in the second minute synchronous events can be observed, but not in the axons in focal plane of the recording, and in the third minute the axons coming from ventral midbrain contribute to a high-frequency synchronous network event which lasts until the end of the recording.

Panel E: To confirm if ventral midbrain axons can functionally interact with neurons in the striatum and cortex, organoids were infected with an AAV containing CAG-hChr2-H134R-tdtomato.

We then used a silicon probe setup (left side of Panel E) and optogenetic activation of the midbrain (right two images of Panel E) to activate neurons in the ventral midbrain and recorded from either the striatum or cortex.

Panel F: Stimulation setup. 10min of baseline in either the striatal or cortical part was recorded. Then, for 10 minutes, the ventral midbrain side was stimulated every 10 seconds for 500ms.

Panel G: example trace of a striatal recording without (blue) and with a 90-250 Hz bandpass filter (black) and the RMS (red). Stimulation started at 615 sec. This recording indicates that neurons from the ventral midbrain make functional connections with neurons in the striatum and cortex. 3/3 recordings in striatum and 3/3 recordings in the cortex displayed similar behavior.

Panel H: Analysis of an individual burst (wideband/bandpass 90-250 Hz, RMS). Note that the burst duration corresponds to the duration of ventral midbrain stimulation (500 ms).

Panel I: Average burst duration of the recording, corresponding to the 500 ms stimulation setup.

Panel J: Optogenetic setup for measuring dopamine release. Organoids were co-infected with an AAV containing Syn-ChrimsonRtdTomato and an AAV containing Syn-GRAB-DA4.4. Thus, stimulation of ventral midbrain dopaminergic neurons should induce dopamine release, which can be measured with GRAB-DA4.4, a fluorescent dopamine sensor.

Panel K: cumulative fluorescence without stimulation in green and cumulative fluorescence with stimulation subtracted from cumulative fluorescence without stimulation in red. Notably, a change in fluorescence was majorly observed on the membrane of cells and neurites (white arrows).

Panel L: Fluorescence change over time of neurons recorded in the striatum. Notably, upon stimulation several regions displayed an up to 10% increase in fluorescence.

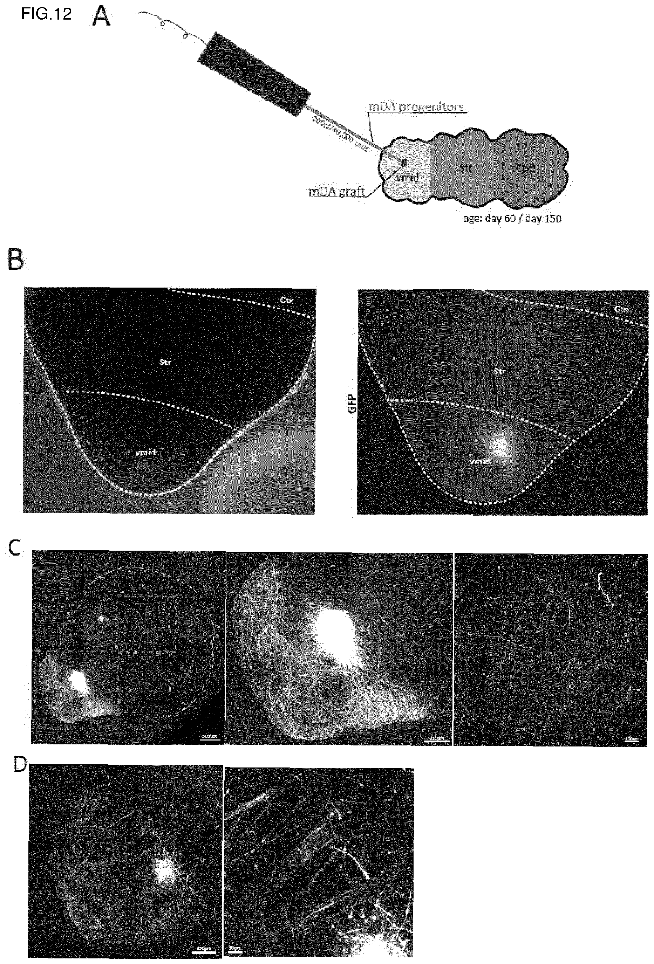

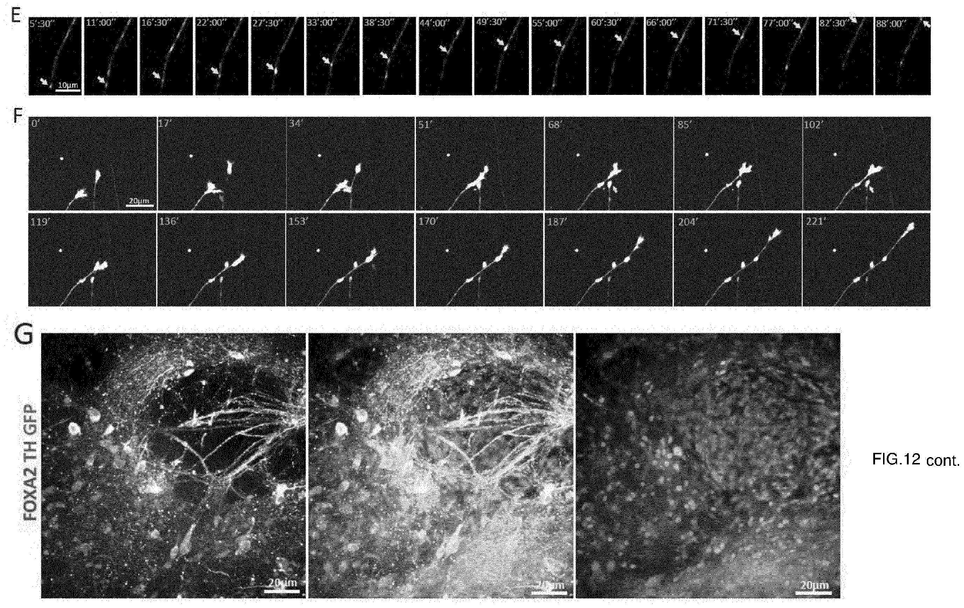

### Figure 12: mDA injections

Panel A: Schematic of the injection setup. Midbrain dopaminergic neuron progenitors (day 16) with endogeneous Cre in the TH locus and infected with a flox-stop-flox GFP lentivirus were grafted into the ventral midbrain side of a triple fusion using a microinjector. 40000 cells in roughly 200 nl were injected in organoids between 60 and 150 days old. Injection of one organoid takes approximately 1-2 minutes.

Panel B: A triple fusion with grafted mDA one month after injection. The graft is easily visible in the ventral midbrain. Panel C: Surface recording of an organoid with 30-day old graft. Notably, the ventral midbrain region is highly innervated, but striatum and cortex also receive significant levels of innervation.

Panel D: dopaminergic bundle formation from grafted neurons, projecting into the striatal tissue.

Panel E: Live-imaging experiment which allows the movement of axonal structures to be visualized, which could be indicative of axonal transport.

Panel F: Live imaging of two dopaminergic axonal growth cones. Notably, one axonal growth cone extends a filopodium or lamellipodium in the direction of the other dopaminergic axon and starts interacting (green arrow). After the left axon continues projecting, a bleb remains at the site of interaction (yellow arrow). This happens a second time with another axon (red arrow). The middle axon does not project further during and after interaction and a bleb is formed at its end.

Panel G: 2ECi recording of an injected graft, immunolabeled for FOXA2 and TH. The grafts form fiber bundles and start projecting.

### Examples:

### Example 1: Material & Methods:

### Example 1.1: Stem cell culture:

The hESC lines (H1 (WAe001-A), H7 (WA07), H9 (WA09), RC17 (RCe021-A)) and in-House generated iPSC lines (176/1, 178/5, 178/6) were cultured feeder-free on growth factor-reduced Matrigel (Corning, Cat.# 354277) in mTESR1 (Stemcell Technologies, Cat.# 85875). Routine genome integrity tests were performed every 10 passages. Cells were split after 3-5 days with avoiding confluently coated wells, in a ratio of 1:6-1:10 using PBS^{-/- 0.5mM EDTA} (PBS: Bio Trend PBS-1A, EDTA: Sigma-Aldrich, Cat.# E6758) and incubated in an incubator until colonies start to break apart (4-8min after incubation).

### Example 1.2: Media formulations:

### -Neural induction media

Media basis: DMEM/F12 (Invitrogen, Cat.# 11330-057), 1% N2 Supplement (ThermoFisher, Cat.# 17502001), 1% GlutaMAX-I (ThermoFisher, Cat.# 35050-038), 1% MEM-NEAA (Sigma-Aldrich, M7145), 1:1000 Heparin solution (Sigma-Aldrich, Cat.# H3149-100KU), 1% PenStrep (Sigma-Aldrich, Cat.# P4333)

### -Improved-A media

Media Basis: 50:50 DMEM/F12:Neurobasal (Gibco, Cat.# 21103049). 0.5% N2 supplement, 2% B27-A (ThermoFisher, Cat.# 12587010), 1:4000 Insulin (Sigma-Aldrich, 19278), 1% GlutaMAX, 0.5% MEM-NEAA, 1% Antibiotic-Antimycotic (ThermoFisher, Cat.#15240062)

### -Improved +A media:

Media Basis: 50:50 DMEM/F12:Neurobasal (Gibco, Cat.# 21103049). 0.5% N2 Supplement, 2% B27+A (ThermoFisher, Cat.# 17504044), 1:4000 Insulin (Sigma-Aldrich, 19278), 1% GlutaMAX, 0.5% MEM-NEAA, 1% Antibiotic-Antimycotic (ThermoFisher, Cat.#15240062), 1% Vitamin C solution (40mM stock in DMEM/F12) (Vitamin C: Sigma-Aldrich, Cat.# A4544), 1 g/liter sodium bicarbonate (Sigma-Aldrich, Cat.# S5761)

### -Brainphys:

Media Basis: BrainPhys Neuronal Medium (Stemcell Technologies, Cat.#05790), 2% B27+A, 1% N2 Supplement, 1 ml CD Lipid Concentrate (Thermo Scientific, Cat.#11905031), 1% Antibiotic-Antimycotic, 1:147 20% Glucose Solution, 20ng/ml BDNF (Stemcell Technologies, Cat.#78005.3), 20ng/ml GDNF (Stemcell Technologies, Cat.# 78057.3), 1 mM db-cAMP (Santa Cruz Biotechnology, Cat.# sc-201567C).

### Example 1.3: Viruses:

AAV8 pAAV-Syn-Archon1-KGC-GFP-ER2 (Addgene Cat.# 115892-AAV8) AAV1 pAAV-Syn-ChrimsonR-tdT (Addgene Cat.# 59171-AAV1) AAV-RG. AAV-CAG-hChR2-H134R-tdTomato (Addgene Cat.# 28017-AAVrg) AAV2 (pAAVss_hsyn-GRAB-DA4.4): 3.61E+13 VG/mL (gift from Boehringer Ingelheim)

### Example 1.4: Antibodies:

| Species | Antigen | Producer | Cat.# | Dilution used* |
|---|---|---|---|---|
| Goat | FOXA2 | R&D Systems | AF2400-SP | 1:300 |
| Goat | OTX2 | R&D Systems | AF1979 | 1:100 |
| Sheep | TH | Abcam | ab113 | 1:400 |
| Rabbit | TH | Abcam | ab112 | 1:300 |
| Rabbit | FoxG1 | Abcam | ab18259 | 1:200 |
| Rabbit | GSX2/GSH | Millipore | ABN162 | 1:100 |
| Mouse | PAX 6 | Abcam | ab78545 | 1:100 |
| Sheep | PAX 6 | R&D Systems | AF8150 | 1:200 |
| Rat | CTIP2/ BCL11b | Abcam | ab18465 | 1:300 |
| Sheep | DLX5 | R&D Systems | AF6710 | 1:100 |
| Goat | SOX2 | R&D Systems | AF2018 | 1:100 |
| Mouse | SOX2 | R&D Systems | MAB2018 | 1:100 |
| Rabbit | SOX2 | Abcam | ab97959 | 1:600 |
| Rabbit | ISL-1 ISL1 Islet | Abcam | AB20670 | 1:100 |
| Goat | DARPP32 | R&D systems | AF6259 | 1:100 |
| Rabbit | DARPP32 | Abcam | ab40801 | 1:100 |
| Rabbit | FOXP1 | Abcam | AB16645 | 1:200 |
| Mouse | GAD67 | Millipore | MAB5406 | 1:200 |
| Rabbit | TBR1 | Abcam | ab31940 | 1:300 |
| Rabbit | Nkx2.1/Thyroid | Epitomics | 6594-1 | 1:1000 |
| | (TTF1) | | | |
| Chicken | GFP | Aves lab/swant | GFP-1020 | 1:500 |
| Rabbit | RFP/tdtomato | Abcam | ab62341 | 1:100 |
| Rat | DAT | Millipore | MAB369 | 1:500 |
| Rabbit | TPH2 | Abcam | ab111828 | 1:500 |
| Goat | Serotonin | Abcam | ab66047 | 1:500 |

| | | | | |
|---|---|---|---|---|
| * For 3D Immunohistochemistry, 5-10x this concentration was used (dependent on antibody quality and tissue size to be stained) Secondary antibodies were the standard panel of Jackson ImmunoResearch and Invitrogen (Alexa Fluor 488, 568 and 647 polyclonal donkey antibodies raised against species of primary antibodies) with the option of affinity-purification where available. | | | | |

### Example 1.5: Generation of cortical organoids:

For cortical organoid preparation, cells were grown for 2-3 days after splitting in MG coated 6-well plates and dissociated using 600µl Accutase (Sigma-Aldrich, Cat.# A6964). Cells were washed off with 1.4ml stem cell medium and spun down at 150g for 5min. Cells were resuspended in 1ml stem cell medium +1:100 RI Y27632 (Selleck Chemicals, Cat.# S1049) and counted. 9000 cells in 150ul of media +1:100RI per well were then transferred into a 96 well ultra-low attachment plate (Thermo Scientific, Cat.# 136101 and Corning, Cat.#7007). After 3 days, 100µl of media was removed and replaced with 150µl of fresh media. On days 5, 7 and 9, 150µl of media was exchanged with 150µl of neural induction media. On day 11, up to 30 EBs were transferred into a 10cm plate which was coated with anti-adherence rinsing solution (Stemcell Technologies, Cat.# 07010). EBs were transferred into 10ml Improved-A media containing 2% liquid Matrigel (Corning, Cat.# 356235) in the media. Notably, media has to be fridge-cold when Matrigel is added and used the same day. Ideally, until EBs are added the media does not reach temperatures above 10°C to prevent Matrigel from polymerizing. On day 12, 3µM CHIR99021 (Merck Millipore, Cat.# 361571) was added to the media. On day 13, media was exchanged with fresh media containing 3µM CHIR99021 and not changed for three days. Organoid media was then exchanged every 3 days, with a change to Improved+A media around day 16. Organoids were transferred to orbital shakers (Inforce Celltron HT, 42RPM) on day 20 and cultured in Improved+A media until day 60, where a gradual shift to Brainphys media (25%, 50%, 75%, 100%) is performed. As soon as organoids were transferred to orbital shakers, media volume was increased to 30ml.

### Example 1.6: Generation of ventral midbrain organoids:

For ventral midbrain organoid preparation, cells were grown for 2-3 days after splitting in MG coated 6well plates and dissociated using 600µl Accutase. Cells were washed off with 1.4ml stem cell media and spun down at 150g for 5min. Cells were resuspended in 1ml stem cell media +1:100 RI and counted. 9000cells were then transferred into 150ul neural induction media containing 200ng/ml Noggin (R&D Systems, Cat.# 6057), 10µM SB431542 (Stemgent, Cat.# 04-0010-10) and 1µM CHIR99021 and ROCK inhibitor in an ultra-low attachment U-shaped 96well plate (Corning Cat#). On day 2, 100µl of this media was removed and replaced with 150µl fresh media of the same formulation.

On Day 4, 150µl of media was exchanged, with the addition of 200ng/ml Noggin, 10µM SB431542, 1µM CHIR99021 and 300nM SAG (Merck, Cat.# US1566660) and 100ng/ml FGF8 (R&D Systems, Cat.# 5057-FF) to the newly added media. On day 6, 150µl of media was exchanged, containing 300µM SAG and 100ng/ml FGF8.

On day 8, organoids were transferred into 10cm plates coated with anti-adherence rinsing solution. Media was exchanged with 12ml Improved-A media containing 2% Matrigel (added to COLD media and used within 1h) and 300µM SAG and 100ng/ml FGF8 for the first feed.

Organoid media was then exchanged every 3 days, with media lacking Matrigel, and with a change to Improved+A media around day 16.

Organoids were transferred to orbital shakers on day 20 and cultured in Improved+A media until day 60, where a gradual shift to Brainphys media (25%, 50%, 75%, 100%) is performed. As soon as organoids were transferred to orbital shakers, media volume was increased to 30ml.

### Example 1.7: Generation of striatal organoids:

For striatal organoid preparation, cells were grown for 2-3 days after splitting in MG coated 6well plates and dissociated using 600µl Accutase. 9000cells were then transferred into 150µl neural induction media containing 10nM SAG and 2.5µM IWP2 (Sigma-Aldrich, Cat.# 10536) as well as 1:100 ROCK inhibitor. Media was exchanged on day 2 by replacing 100µl media with 150µl fresh NI media containing 10nM SAG, 2.5µM IWP2 and 1:100 ROCK inhibitor. On day 4, media was exchanged again by replacing 150µl media with 150µl fresh NI media with 10nM SAG and 2.5µM IWP2. On day 6, 150µl of media were exchanged with NI media without factors. On day 8, organoids were transferred into 10cm plates coated with anti-adherence rinsing solution. Media was exchanged with 12ml Imp-A media containing 2% Matrigel (added to COLD media and used within 1h). Organoid media was then exchanged every 3 days, with media lacking Matrigel, and with a change to Improved+A media around day 16. Organoids were transferred to orbital shakers on day 20 and cultured in Improved+A media until day 60, where a gradual shift to Brainphys media (25%, 50%, 75%, 100%) is performed. As soon as organoids were transferred to orbital shakers, media volume was increased to 30ml.

### Example 1.8: Generation of ventral hindbrain organoids:

For ventral hindbrain organoid preparation, cells were grown for 2-3 days after splitting in MG coated 6well plates and dissociated using 600µl Accutase. Cells were washed off with 1.4ml stem cell media and spun down at 150g for 5min. Cells were resuspended in 1ml stem cell media +1:100 RI and counted. 9000cells were then transferred into 150µl neural induction media containing 2µM DMH1 (R&D Systems, Cat.# 4126), 2µM SB431542 and 1.4µM CHIR99021 and ROCK inhibitor in an ultra-low attachment U-shaped 96well plate. On day 2, 100µl of this media was removed and replaced with 150µl fresh media of the same formulation.

On Day 4, 150µl of media was exchanged, with the addition of 2µM DMH1, 2µM SB431542, 1µM CHIR99021, 300nM SAG and 50ng/ml FGF4 (R&D Systems, Cat.# 7460-F4-025/CF) to the newly added media. On day 6, 150µl of media was exchanged, containing 300µM SAG and 50ng/ml FGF4.

On day 8, organoids were transferred into 10cm plates coated with anti-adherence rinsing solution. Media was exchanged with 12ml Imp-A media containing 2% Matrigel (added to COLD media and used within 1h) and 300µM SAG and 50ng/ml FGF4 for the first feed.

Organoid media was then exchanged every 3 days with media lacking Matrigel, and with a change to Improved+A media around day 16.

Organoids were transferred to orbital shakers on day 20 and cultured in Improved+A media until day 60, where a gradual shift to Brainphys media (25%, 50%, 75%, 100%) is performed. As soon as organoids were transferred to orbital shakers, media volume was increased to 30ml.

### Example 1.9: Generation of PDMS fusion embedding molds:

Embedding molds have been designed in Tinkercad (www.Tinkercad.com) and were adjusted in diameter and length based on organoid size on the day of fusion. Files were exported as .stl files and loaded into the slicer software XYZ print 1.4.0. The negative was printed using transparent PLA with 100% infill density and 0.1mm layer height and 215°C nozzle temperature. After printing, the molds were treated with a Heatgun (Bosch Hot Air Blower 1800W) at 550°C to carefully melt the surface of the mold, creating a smooth finish and removing the typical rough surface of 3D printing.

The positive was then casted using polydimethylsiloxane (PDMS). In brief, 5ml of curing agent and 45ml of Monomer (both Sylgard^{®} 184 Elastomer Kit, VWR) were intensively mixed. The mixture was then spun down to remove air bubbles and used directly.

To reduce the extent of bubbles formed during curing, the molds were first cast at room temperature (24°C). For this, the 3D printed negative is placed in a 10cm plate with the wells looking up. The PDMS was then carefully poured on the middle of the mold and the plate is filled with approx. 45ml of PDMS. After pressing the negative down to remove air bubbles, the molds were cured overnight at RT. For faster curing, the mold was alternatively transferred into a 55°C incubator, however this can increase the release of gas and thus bubble formation into the PDMS. When curing at RT overnight, the cast was transferred to 55°C for 2-3 hours the next day to finalize curing.

The mold was then cut out of the plate and washed in 70% Ethanol for 30min and then dried.

The negative was re-used as it is not damaged in the casting procedure.

### Example 1.10: Generation of mold assisted fused organoids:

Molds were coated in an anti-adherence solution to increase the non-stick behavior of PDMS further. After coating, the molds were washed once in PBS.

For linearly fusing organoids, organoids were transferred group by group into the embedding molds, with up to 32 fusions at once. For this, the first group (exemplary cortical organoids) was transferred into all molds first, transferring as little media as possible with the organoid. Secondly, striatal organoids were transferred to the right of the cortical organoids, and third, the procedure was repeated with ventral midbrain organoids. Optionally, ventral hindbrain organoids were added as well.

After transfer, residual media of the organoids was removed entirely, paying close attention to not disturbing the positioning of the different organoids. This step allows the attachment of organoids linearly (without media, organoids become sticky and attach to each other). After roughly 30sec- 1 minute, a small amount of liquid Matrigel (about 15µl) is added to the fusions and the fusions were transferred into an incubator (37°C, 5% CO2, humidified) for 20min. After this incubation step, the linear fusions can be easily washed out of the embedding molds. The embedding molds can be directly used for fusing organoids again or washed in PBS and 70% Ethanol (cell-culture grade) and then dried.

Fused organoids were cultured in 10cm plates without shaking for the first 2 days, before being transferred to an orbital shaker in 30ml of media with reduced shaking speed (Inforce shakers, 42rpm instead of 56rpm).

### Example 1.11: scRNAseq protocol:

Organoid fusions were separated by a scalpel. Ventral midbrain, striatal and cortical tissue were processed separately. Before dissociation, necrotic material from the core of the organoid was washed off in PBS^{-/-}. Organoids were then transferred into a 1.5ml Eppendorf tube and all PBS was removed. 1ml of Trypsin-Accutase (1:10) (Trypsin: Thermo Fisher, Cat.# 15090046) was added to the organoids and the tubes were transferred to a tube shaker at 37°C 700RPM. Tubes were flipped every 3-4 minutes until the tissue was dissolved, or for a maximum of 40min. After dissociation, 500µl of ice-cold PBS^{-/-} were added on top. Cloudy material on top of the solution, which forms occasionally, can be removed with a pipet. Tubes were then spun down in a precooled (4°C) table centrifuge at 400rpm for 5min. Then, as much liquid as possible was removed without damaging the cell pellet. Pellets were carefully resuspended in 400ul of PBS^{-/-} and filtered through a 40µm cell strainer.

Cells were then counted. Live cell count had to be above 80% and above a concentration of 200.000cells/ml, but not more than 2.7mio cells/ml. Samples were diluted down, where needed. Multiseq labeling using MULTI-seq Lipid-Modified Oligos was performed directly after counting following the manufacturer's protocol (Sigma-Aldrich, Cat.# LM0001). In brief, 40ul of anchor solution including barcode was mixed with the sample and incubated on ice for 5min. 40µl of co-anchor were then added and mixed and incubated on ice for 5min. The reaction was then topped up with 1ml of ice-cold PBS^{-/-} containing 2% BSA, and mixed to bind residual anchor and co-anchor.

Cells were spun down at 400-600RPM for 5min at 4°C. The supernatant was removed and cells were resuspended in 200µl PBS^{-/-} with 2% BSA and supplemented with 0.8µg/ml DAPI.

Cells were then FACS sorted for live cell sorting (DAPI⁻) in a FACS Aria III machine and a 70µm nozzle (approx. 1nl per cell) into a 1.5ml Eppendorf tube with 20µl of PBS^{-/-} and 2% BSA. Library preparation was performed using the service of the Vienna BioCenter NGS facility using a 10x Genomics stranded kit and NovaSeq S1 Asymmetric 10X Illumina sequencing.

### Example 1.12: scRNAseq analysis:

scRNAseq analysis was performed using R Studio and the Seurat toolset of the Satija lab (satijalab.org/seurat/). Cutoffs for cells were defined as mRNA (20%), feature_RNA (6000) and ribosomal 40%.

### Example 1.13: Cryoprocessing of tissue:

Organoid tissue was rinsed once in PBS and fixed in 4% Formaldehyde solution in 1× PBS for 4h at RT. The tissue was then incubated for one day in 30% sucrose at 4°C. Tissue is subsequently transferred to a bed of OCT (Scigen, Cat.#4586) and OCT is carefully swirled around the organoid. After 5min incubation, organoids were transferred into cryomolds and transferred onto a metal block on dry ice. Once a small ring of solidified (=frozen) OCT appears, the cryomolds were filled up with OCT and were completely frozen. The resulting cryoblocks were then wrapped in aluminum foil and were transferred to at least -70°C until cryosectioning.

Frozen organoid tissue was sliced into 20µm (regular organoids) or 30µm (for better representation of axons) using a cryostat, and collected on cryoslides.

Sections were dried for at least 3h before processing for immunostaining, or alternatively dried overnight and then stored at -20°C.

### Example 1.14: 2D IHC:

For Immunohistochemical fluorescent labeling, slides were thawed and dried for 1h at RT. Residual OCT was then washed off by washing 5min in PBS on an orbital shaker. Permeabilization/blocking was performed by gently dropping PBS containing 5% BSA and 0.3% TX100 (Sigma-Aldrich, Cat.#93420) on the slides and incubating them at RT in a humidification chamber for 30min. Permeabilization/blocking solution was sterile filtered and then frozen, and fresh aliquots have been used for every staining round.

Primary antibodies were added at desired dilutions in staining solution (5% BSA, 0.1% TX100, in PBS, sterile filtered and frozen until needed) and incubated at 4°C overnight in a humidification chamber. On the second day, the slides were rinsed 3x with PBS and then washed 3x for 10min in PBS-T (PBS+ 0.1% TX100) at RT on an orbital shaker. Secondary antibodies were added at a 1:500 dilution and slides were incubated for 2h at RT in a humidification chamber. DAPI (2µg/ml in PBS) was added for 7min, then slides were rinsed 3x in PBS and then washed 2x in PBST. The last washing step was performed using only PBS. Coverslips were mounted using fluorescent mounting medium (DAKO, Cat.# S3023). Slides were stored at RT for at least 4h before using for microscopy and at 4°C for storing.

### Example 1.15: 3D IHC:

2ECi tissue clearing was performed as previously described (Masselink & Reumann, et al., Development 146, 2019). In brief, organoids were fixed with 4% formaldehyde in PBS for 4h at RT. For 3D immunohistochemistry, PBS-TxDB was prepared: 10x PBS, 5% BSA and 2% TX100 were pre-mixed and filled up with distilled water to achieve approx. 70% of aimed volume (e.g. for 11 of PBS-TxDB, 100ml of 10x PBS, 50g BSA and 20ml TX100 were used and filled up to 700ml with distilled water. 20% DMSO was then added slowly under heavy stirring - rapid addition of DMSO may result in irreversible BSA aggregation. The solution was filled up to 100% with distilled water and sterile filtered. PBS-TxDB is stable at RT for at least several weeks, or at 4°C for up to a year.

Organoids were blotted/permeabilized on a rotor or shaking plate for 1-2 days in 10ml PBS-TxDB at RT. Organoids (dependent on the size, between 3 and 6) were then transferred into 2ml Eppendorf tubes containing primary antibody solution in PBS-TxDB (between 500µl to 1ml) and transferred to a rotor or shaking plate for 5 days. Organoids were then washed in 10ml PBS-TxDB for 2 days (1x rinse, 3x PBS-TxDB exchange) and the same steps were repeated for secondaries. After washing off the residual secondaries, organoids were washed in PBS for 1h and then fixed in 4% Formaldehyde solution for 30min-2h. Organoids were then ready to be dehydrated in a 1-Propanol gradient (30%, 50%, 70%, 100%, 100%, 4-8h per step). As no endogenous fluorescence had to be maintained, pH adjustment of the 1-Propanol was skipped. After dehydration, organoids were transferred into Ethyl Cinnamate and were ready for imaging as soon as completely transparent (1-3h or overnight at RT). Cleared organoids were stored at room temperature and should not be transferred to 4°C, as Ethyl Cinnamate crystallizes out at lower temperatures.

### Example 1.16: Microscopy (2D, 3D, timelapse):

Cell culture microscopy was performed using a Zeiss Axio Vert.A1 widefield microscope with the Axiocam ERc 5s camera (Zeiss GmbH).

2D and 3D tissue clearing recordings were performed using an Olympus Spinning Disk system based on the Olympus IX3 Series (IX83) inverted microscope. The system is equipped with a dual-camera Yokogawa W1 spinning disk using 405nm, 488nm, 561nm and 640nm lasers and recorded with the Hamamatsu Orca Flash 4.0 camera. Objectives used were 10×/0.3 (Air) WD 10mm, 10×/0.4 (Air) WD 3.1mm, 20×/0.75 (Air) WD 0.6mm and 40×/0.75 (Air) WD 0.5mm. For live imaging, the attached incubator setup was used (37°C, 5% CO2). For high magnification live imaging with air objectives, the additional 3.2x magnification of the Orca Flash 4.0 camera was used.

For dopamine sensor live imaging, a Visiscope Spinning Disc Confocal (Visitron SystemsGmbH) was used. This system is based on a Nikon Eclipse Ti E inverted microscope, equipped with a Yokogawa W1 spinning disc and an incubator setup (used at 37°C, 5% CO2). Components are controlled by the Visiview software. Lasers used were 405nm 120mW, 488nm 200mW, 561nm 150mW and 640nm 150mW using the Andor Ixon Ultra 888 EMCCD camera (13µm pixel, 1024x1024 pixels) or the PCO Edge 4.2m sCMOS camera (6.5µm pixel, 2024x2024 pixels).

### Example 1.17: Image processing:

Images were processed using the FIJI distribution of the open-source image processing application ImageJ (version 1.53c). For 3D reconstruction, the open community software Icy was used (icy.bioimageanalysis.org/).

### Example 1.18: GCAMP setup:

Organoids were grown until day 120. GCAMP was recorded using an Olympus Spinning Disk (see Microscopy section). In brief, 20x magnification and an exposure time of 65ms per frame (continuous recording) were used for up to 6.5min of continuous recording. For trace extraction, recordings were scaled down from 2048x2048 to 512x512pixels. Trace extraction was performed using the open-source software package CaImAn (Flatiron Institute).

### Example 1.19: Extracellular recordings and optogenetic stimulation:

Organoids were transferred in a batch recording chamber (PC-41 LP, Warner Instruments) and mounted with small amounts of 4% agar in Phosphate Buffer (PB). The recording chamber was then transferred to a pre-warmed dish incubator (DH-35iL, Warner Instruments) and perfused with 5ml/min (95% O2 5%CO2 infused) ACSF at 37°C (controlled by TC-344C, Warner Instruments) through a peristaltic pump (PPS2, Multichannel System). For extracellular spike detection, signals were recorded using silicon probes with two shanks and 16 recording sites each (total of 64 recording sites, Probe ASSY-77 P-2, Cambridge NeuroTech). Probes were reused for recordings and were washed in 1% Tergazyme (Merk, Cat.# Z273287) in MilliQ water for 30min at 60°C. Probes were then washed with iso-Propanol and rinsed in MilliQ water.

Probes were positioned on the organoids and acquisition was performed at 16bit resolution on a 64-amplifier chip (RHS2000, Itan Technologies) with the open-source tool open-ephys. The signal sampling rate was set to 30kHz per channel and filter to 0.1Hz high-pass and 5kHz.

For optogenetic stimulation, a light source (Sola SMII, Lumen-cor) was connected to a collimator with insertable emission filters (630/69nm and XXX, Thorlabs). From there, the filtered light was transported with a fiber optic glass (400µM, NA=0.39, Thorlabs) to the organoid. For stimulation, the fiber optic glass was positioned less than 1mm away from the site of stimulation.

Pulses were generated using Pulse Pal v2 (Sanworks).

Prior to recording, the probes were positioned on the organoid and incubated for 5min. Then, a 10min baseline recording was performed. Subsequently, the organoids were stimulated for 10min following a 500ms-10sec interval (500ms stimulation, 10sec pause).

Electrophysiology analysis was performed using the "minibrain" pipeline (github.com/JoseGuzman/minibrain) by Jose Guzman.

### Example 1.20: Viral transduction protocol:

For individual organoids, 1×10¹³ vg was used. For triple fusions, 3×10¹³ were used. Viral titer might have to be significantly adjusted based on organoid size, or transduction efficiency of the virus.

Organoids were transferred into 24 well plates and were incubated with the virus in 400µl of media for 4h. Subsequently, organoids were transferred into 6cm plates together with the media containing the virus. Organoids of the same group can be transferred together. Media was then added to achieve a volume of 6ml (individual organoids) or 7ml (fusions).

Organoids were transferred to an orbital shaker overnight. The next day, organoids were transferred back into 10cm plates containing 30ml of media.

### Example 1.21: qPCR:

For each condition, 6-8 organoids were collected at indicated time points into 2ml RNAse-free tubes. RNA was extracted using the RNeasy mini kit (Qiagen). cDNA synthesis was performed using 1µg of total RNA and Superscript II (Invitrogen) enzyme, following protocols provided by the manufacturer. qPCR was performed using Sybr Green master mix (Promega) on a BioRAD 384-well machine (CXF384). The protocol used is: (1) 95°C 3min, (2) 95°C 10sec, (3) 62°C 10sec, (4)72°C 40sec, (5) go to 2, repeat 40x, (6) 95°C lmin, (7) 50°C 10sec.

Quantification was performed in excel by calculating ΔCt relative to TBP. Data are represented as expression level (2^{-ΔCt}) relative to TBP.

### Example 1.22: Statistical analysis:

For statistical analysis, GraphPad Prism 8.1.1 was used.

### Example 1.23: mDA graft injections:

16-day old mDA progenitors were thawed in a water bath until a small sliver of ice is remaining. 500µl of room temperature DMEM/F12 with 20% KOSR (ThermoFisher, Cat.# 10828028) and 1:100 RI was added drop by drop on top of the cells. Cells were then transferred into 10ml of DMEM/F12 with 20% KOSR and 1:100 RI and spun down at 500g for 5min. The supernatant was removed and cells were resuspended in exactly 1ml of media and counted. Cells were then spun down again and resuspended in the required volume for injecting (20.000 cells per 100nl of media).

Organoids were injected using a Nanoinjector (Nanoject II, Drummond) and pulled glass capillaries (Drummond^{™} Capillaries for Nanojet II^{™} injectors) which were pulled using a Micropipette puller (Model P-97, Sutter Instrument). A total of 207nl was injected in three pulses (69nl each) in the fast injection mode. Prior to injection, organoids were transferred into an empty plate and all media was removed. After injection, organoids were not touched for one minute to allow closure of the injection site. Organoids were then transferred back into 10cm plates with media and incubated without shaking for one day, before being transferred back to an orbital shaker.

### Example 2: Recreation of dopamine-associated brain regions in cerebral organoids

The human dopaminergic system is majorly comprised of dopaminergic neurons in the ventral midbrain. From there, two major dopaminergic cell types project into the striatum and cortex. The first group of dopaminergic neurons are A9 dopaminergic neurons in the substantia nigra compacta. A9 dopaminergic neurons are associated with the nigrostriatal pathway and project into the dorsolateral striatum, where they have a crucial function in fine motor control (Arenas, Denham and Villaescusa, Development 142, 1918-36, 2015). These A9 dopaminergic neurons preferentially degenerate in Parkinson's disease (Lees, Shin and Revesz, Lancet 373, 2055-66, 2019). The other, more abundant population of dopaminergic neurons are A10 dopaminergic neurons from the ventral tegmental area. A10 dopaminergic neurons project to the ventral striatum as well as into the cortex, majorly into the limbic system and prefrontal cortex. A10 dopaminergic neurons are associated with affective encoding ("dopaminergic reward pathway") (Lio, Shin and Ikemoto, Neuropsychopharmacology 33, 2182-94, 2014).

During development, dopaminergic axons first send out axons dorsally and are then attracted rostrally. They then project longitudinally through the midbrain and diencephalon and form the medial forebrain bundle (MFB). Once dopaminergic axons reach the telencephalon, they particularly innervate the striatum and project further into the developing cortex (Arenas, Denham and Villaescusa, Development 142, 1918-36, 2015).

While the dopaminergic system is relatively well understood in rodents, dopaminergic neurons project into the arguably evolutionary most different regions in the human brain: striatum and cortex (Florio and Huttner, Development 141, 2182-94, 2014) (Balsters et al., Elife 9, 1-24, 2020). Additionally, rodents generally do not develop Parkinson's disease, which makes artificial lesion models necessary (Stroker& Greenland, Chapter 5 Table 3, 2018).

The targeted regions and the connectome of both A9 and A10 dopaminergic neurons are relatively well understood, however, not much is known about the factors which contribute to A9 or A10 differentiation, as well as why A9 dopaminergic neurons are so much more affected in Parkinson's disease.

We thus decided to work on an in vitro system to be able to model human-specific aspects of the dopaminergic system. Such a system would ideally include the generation of a diverse population of dopaminergic neurons from the ventral midbrain, but also its interaction partners (dopamine sensitive neurons) in the striatum and the cortex. Additionally, such a system should be scalable and allow for the formation of dopaminergic circuits to be studied on a functional level.

While many axonal outgrowth models exist (e.g. coated surfaces), we decided to aim for a 3D environment, which should also provide more appropriate chemotactic signaling and an environment more similar to that *in vivo.* We also wanted to challenge this system by investigating if ventral midbrain dopaminergic grafts would behave similarly to the *in vivo* situation and innervate their target regions.

To achieve the goal of a 3D model system for the ventral midbrain, we used brain organoids. Brain organoids depict the early stages of neurogenesis, but also allow us to study the maturation of neurons and their properties, for example by looking at neuronal activity. Additionally, several attempts have been made to study brain region interactions using fused brain organoids, such as in the example of pallial::subpallial fusions (Bagley et al., Nat Methods 14, 743-751, 2017) (Birey et al., Nature 545, 54-59, 2017) and cortex::thalamic fusions (Xiang et al., Cell Stem Cell 24, 487-497, 2019).

As a first step, we tried to recreate the three major regions which are associated with A9 and A10 projections: ventral midbrain, striatum and cortex.

As cortical organoids are already a well-established protocol and many publications exist which use cortical organoids (Giandomenico et al., Nat. Neurosci. 22, 669-679, 2019, Eichmüller et al., Science 375, 6579, 2022), we developed patterning protocols for the ventral midbrain (Figure 1A, 2, Figure 3) and striatum (Figure 1A, 4, Figure 5) by patterning organoids along the anterior-posterior axis and dorsoventral axis (Figure 1B).

To test the versatility of the method, we further developed a novel protocol for the generation of ventral hindbrain organoids which contain serotonergic neurons. Serotonergic neurons, similar to dopaminergic neurons, broadly innervate forebrain structures and are vital for, among others, mood control, reward and learning. Dysfunctions in the serotonergic pathways are associated with a range of diseases such as depression and Amyotrophic Lateral Sclerosis (ALS), but also many Parkinson's disease (PD) patients show a decline in serotonergic neurons, which is associated with the non-motor symptoms of PD (anxiety, depression, dementia, sleep disturbances).

### Example 3: Ventral midbrain organoids

Ventral midbrain dopaminergic neurons are born at the caudal floor plate of the ventral midbrain, close to the hindbrain (Arenas, Denham and Villaescusa, Development 142, 1918-36, 2015). For the generation of ventral midbrain protocols, we modified existing protocols from 2D and 3D differentiation approaches (Jo et al., Stem Cell 1-11, 2016, Nolbrant et al., Nat Protoc 12, 1962-1979, 2017). In a dual smad inhibitory context, we first defined the amount of sonic hedgehog activation using the small molecule SAG (Figure 2A) and found that a range from 200-1000µM reliable had a floor plate induction effect, as confirmed by qPCR. Preferably we used 300nM SAG. The addition of FGF8 was additionally performed to control for slight off-patterning and increase mDA yield, as described before (Arenas, Denham and Villaescusa, Development 142, 1918-36, 2015).

For Wnt activation as posteriorizing factor, we found a concentration of 1µM to be sufficient for midbrain induction (Figure 2 B-D, the forebrain and midbrain marker OTX2 is still expressed, the dopaminergic marker TH is still expressed). Immunolabeling confirmed the presence of these markers and thus confirms that the modified protocol using SAG300nM CHIR1.0µM produces ventral midbrain dopaminergic neurons (Figure 2E-G). We additionally confirmed the robustness of the protocol by using 4 cell lines with two different genetic backgrounds and origin (176-1: iPSC, H9: hESC, Figure 3C-E).

To summarize, we are able to grow ventral midbrain organoids with the ability to produce dopaminergic neurons.

### Example 4: Striatal organoids

Striatal neurons are born majorly from the lateral ganglionic eminence (LGE), from where they migrate and form the developing striatum (Fjodorova, Noakes and Li, Neurogenesis, 2(1), 2015, Ornorati et al., Nat. Neurosci. 17, 1804-15, 2014). To pattern into LGE, we performed a dose curve experiment in a Wnt inhibitory context (IWP-2, which anteriorized and inhibits dorsal fate) similar to previous 2D differentiation approaches (Wu et al., Strem Cell Reports 11, 635-650, 2018). In brief, we activated sonic hedgehog signaling with the small molecule SAG at concentrations between 10nM and 100nM (Figure 4B). Notably, already the lowest concentration of SAG resulted in a high level of rosettes expressing the LGE marker GSX2 (Figure 4C) while having a low number of rosettes expressing the more dorsal marker PAX6 (Figure 4D). While SAG25nM had similar numbers, we noticed that the striatal (and cortical) marker CTIP2 was already significantly lower in SAG25nM treated organoids (Figure 4E, F).

We additionally performed immunolabeling experiments for a range of striatal progenitor and young born striatal neuron markers, among others SOX2, ASCL1, DLX5 and ISLET1 (Figure 4G-J). We additionally performed this protocol in a range of cell lines (both iPSCs and hES cells) and could confirm the existence of GSX2+ rosettes in all but one cell line (Figure 5B (exemplary), Figure 5C). However, as levels of endogenous SHH signaling might vary in each cell line, different concentrations of sonic hedgehog activation (or even inactivation) might be needed to achieve LGE induction.

Next, we looked at marker expression of typical striatal neurons. Striatal neurons express the marker DARPP32, which labels dopamine-sensitive cells (Figure 4L) and is broadly expressed in the striatum, as well as FOXP1, another striatal marker (Figure 4N) (Arlotta et al., J. Neurosci. 28, 622-632, 2008). Additionally, we investigated if DARPP32⁺ cells also start to become GABAergic, and indeed on day 80 the majority of DARPP32⁺ cells expressed GAD1, a marker for GABAergic neurons.

We could confirm the expression of DARPP32 in multiple cell lines (Figure 5 C-F).

We additionally found multiple cells in striatal organoids expressing Nkx2.1, one of the markers of MGE-derived cells, indicating that not just striatal medium spiny neurons, but also interneurons were produced in striatal organoids (Figure 5 G).

To summarize, we are able to grow striatal organoids with the ability to produce striatal neurons.

### Example 5: Ventral Hindbrain Organoids

Serotonergic neurons of the raphe nuclei, similar to dopaminergic neurons, emerge from the ventral part of the neural tube and are part of the raphe nuclei, from where they project to almost the entire central nervous system (Ren et al., Elife 8, 1-36, 2019). For the generation of a ventral hindbrain protocol, we modified an existing protocol for the generation of 2D striatal neurons (Lu et al., Nat Biotechnol 34, 89-94, 2016). Using two small molecules for DualSmad inhibition (DMH1 and SB431542) and a higher concentration of CHIR (1.4µM) from day 0 until day 6, we patterned into the hindbrain (Figure 1A). On day 4 we applied the small molecule SAG at 300nM, together with FGF4, as both activation of SHH and FGF4 signaling have been shown to activate the serotonergic program (Ye et al., Cell, 93, 755-66, 1998). Using this protocol, we could achieve organoids which were comprised of serotonergic neurons (TPH2 and 5-HT positive, Supplemental Figure 6B).

Thus, we were able to generate a protocol which allows us to study features of ventral hindbrain serotonergic neurons in 3D neuronal culture.

### Example 6: Organoid fusion

We next developed a robust method to bring cortical, striatal and ventral midbrain organoids together. For this, we thought of a system which would allow us to position the organoids in their anterior-posterior orientation, or in the sequence dopaminergic neurons start to innervate. We developed PDMS linear fusion molds, which allow the positioning of three, or more, organoids in a linear manner (Figure 6A, B). To do so, we first created a CAD designed negative which was printed using a 3D printer with PLA filament. We then cast the positive using Polydimethylsiloxane (PDMS) (Figure 6A).

For linearly fusing organoids, we positioned the cortical, striatal and ventral midbrain (and, in the case of quadruple fusions, the ventral hindbrain) one by one in a row into the molds. We then removed the media and waited for at least 30 seconds to let the tissues stick to each other, before adding a small droplet of Matrigel. After polymerization, the linearly fused organoids were gently washed out of the mold with Improved+ A media.

The resulting tissues allowed us to observe that the different organoids nicely grew together over time (Figure 6C). Notably, over several batches we achieved a fusion efficiency of roughly 98%.

By the introduction of a constitutive GFP (CAG-GFP) expressing cell line into the ventral midbrain, we could observe first axonal outgrowth and axon bundles forming already 12 days after fusion (Figure 6D), with an increase of innervation over time to densely innervated striatal and cortical regions (Figure 6E). We could additionally confirm by immunolabeling that the majority of axons from the ventral midbrain were of dopaminergic nature (Figure 6F, G) and that these dopaminergic axons innervated both TBR1⁺ (cortical) as well as DARPP32⁺ (striatal) regions.

By growing Cortex(GFP)-Striatum(unlabeled)-ventral Midbrain (tdtomato) fusions, we could additionally confirm that striatal tissues were not just innervated by ventral midbrain, but also by cortical axonal projections.

To summarize, we found a highly efficient and scalable method to fuse organoids in a spatially controlled manner, which is of essence if interactions with more than two regions have to be observed.

### Example 7: ScRNAseq

To investigate tissue identities in triple fusions, we performed single-cell RNA sequencing of day 60 linear fusions. To be able to separate cells from different regions unbiased from their expression profile, we separated the cortical, striatal and ventral midbrain tissues of fusions using a scalpel and labelled cells coming from the cortex, striatum or ventral midbrain individually using Multi-seq.

We sampled roughly 1000-1800 cells per region (Figure 8A). While the separation of the regions naturally is not perfect and there was always overlap between the different regions, we could see that cortex, striatum and ventral midbrain mostly clustered separately from each other (Figure 8B).

We next performed analysis of marker expression and found clusters of cortical, striatal and ventral midbrain neurons (Figure 8C). Additionally, we found a separate cluster of MGE-derived interneurons, as well as the first astrocytes which were emerging.

Both apical and basal progenitors could be found in the dataset (Figure 8 E', E").

Cortical neural progenitors readily expressed the markers SOX2, PAX6, FOXG1 and EMX2. Interestingly, we found a cluster of EOMES positive cortical intermediate progenitors which was more closely clustering with cortical neurons (FOXG1, EMX1, TBR1, SLC17A7) (Figure 8D).

Striatal progenitors are positive for FOXG1 and ASCL1, and, when terminally differentiating, start to express GAD1, GAD2, FOXP1, BCL11B and ZFHX3 (Figure 8E). The striatum is comprised of more than 85% spiny projection neurons (SPNs), and while mature medium spiny neuron markers such as dopamine receptor type 1 and 2 (DRD1, DRD2, or D1 and D2) were not broadly expressed on day 60, we could indeed find markers of D1 (EBF1, ISL1, TAC1) and D2 specific (SP9, SIX3, GIRK3) differentiation (Figure 8E‴).

We next checked ventral midbrain specific markers and found FOXA2 positive cells, which also expressed key markers of dopaminergic differentiation; amongst others LMX1B, EN1 and PITX3, as well as TH (Figure 8F). Surprisingly, we only found a small number of TH⁺ neurons, which we speculate could be due to the fact that dopaminergic neurons are highly fragile and hard to dissociate, and improved protocols might be needed to increase the fraction of dopaminergic neurons.

We next investigated the remaining clusters in the data set and found that one cluster expressed genes typical for medium ganglionic eminence (MGE) derived interneurons, such as Nkx2.1, LHX6 and SOX6 (Figure 8G). We additionally found first cells being positive for astrocyte markers (GFAP, S100B) or oligodendrocyte markers (OLIG1, OLIG2), indicating that the switch from neurogenesis to astrogenesis and oligodendrogenesis already occurred in a small population of neural progenitors (Figure 8H). We also observed a population of neurons from ventral midbrain organoids being positive for GABAergic markers, hinting at the existence of midbrain GABAergic neurons in ventral midbrain patterned organoids (Figure 8C, "vMid GABA").

This dataset confirms that we have all required cells of the dopaminergic system in the linear fusions and indicates that day 60 is a good time point to study neurogenesis of all three regions, as progenitors and neurons are still broadly present.

### Example 8: 2ECi tissue clearing allows morphological reconstruction of dopaminergic neurons and shows maturation features of dopaminergic axons

We next tried to recapitulate morphological aspects of the dopaminergic circuit and used a recently published tissue clearing protocol to perform whole-mount immunolabeling and 3D recordings of dopaminergic neurons and their projections.

We could find that dopaminergic neurons usually aggregated in clusters (Figure 9A, G), a phenomenon which can be found in vivo but to our knowledge has not been described in vitro. Additionally, we could confirm that both striatal and cortical regions were highly innervated by dopaminergic axons (Figure 9A') and that dopaminergic axons often formed axon bundles (Figure 9B, C), which sometimes were more than a millimeter in length (Figure 9B).

Axonal boutons are the sites where synapses typically occur, which makes them an important metric for structural neuronal maturation. We noticed that between day 40 and 120 the number of axonal boutons on dopaminergic axons drastically increased (Figure 9D, E) to more than 3 axonal boutons/10µm on average on day 120. While the striatum had significantly more axonal boutons on day 40, at later timepoints the density equalized out between both striatum and cortex.

We could also notice that axons generally avoided regions of neurogenesis (Figure 9F), indicating the existence of chemotactic repellents similar to *in vivo.*

Dopaminergic neurons have different morphologies dependent on their location and function in the brain, although still poorly described in the developing human brain. However, we found that dopaminergic neurons clustered together (Figure 9A, G) and displayed a variety of morphologies (Figure 91), indicating a heterogeneous population of dopaminergic neurons. Surprisingly, we also found a TH⁺ population in striatal and cortical organoids. As previous reports exist of TH positive interneurons, we grew fusions containing a DLX56i-GFP interneuron reporter cell line in the striatal part (which we speculated would be the only interneuron producing region). Co-staining for GFP and TH could indeed confirm that TH+ cells in the striatum and cortex were of interneuron origin (Figure 9H).

As another investigation of dopaminergic maturation, we immunolabeled for both TH and dopamine transporter (DAT) in 2D cryo-sectioned triple fusions. We could find that dopaminergic neurons in the ventral midbrain (Figure 9J) expressed DAT, but also dopaminergic axons in the striatum (Figure 9K) and cortex (Figure 9L). DAT is vital for dopamine transport and indicates that dopaminergic neurons matured to a functional level, releasing and re-uptaking dopamine from its synapses.

By fusing three fusions with a constitutive GFP expressing striatal organoid (CAG-GFP), we could see that there were reciprocal connections from the striatum to cortex and ventral midbrain, and by immunolabeling for GABA we could confirm that some of these projections were positive for GABA, confirming that there are GABAergic long-range connections in the fusions - one of the features of striatal projection neurons (Supplemental Figure 6A). Notably, these GABAergic projections could be found in both the striatum and cortex, but while in the ventral midbrain usually only GFP+ axons could be observed, many cells in the cortex were GFP and GABA double positive, indicating migrated interneurons. Thus, we speculate that many GFP GABA double positive axons in the cortex are not of striatal projection neuron identity, but of interneuron identity.

To summarize, these experiments show that we can recapitulate morphological features of the dopaminergic system to an unprecedented level and that reciprocal connections form between Ctx::Str::vMid fusions.

### Example 9: Addition of ventral hindbrain organoids allows the study of serotonergic pathways

To further test the fusion system, we developed a protocol for ventral hindbrain organoids containing serotonergic neurons (Figure 10B) and made linear fusions composed of Cortex::Striatum::ventral Midbrain::ventral Hindbrain fusions using our previously developed fusion molds. We used a cell line with constitutive GFP expression to label the ventral hindbrain (Figure 10C). We could observe high levels of innervation in the midbrain, with medium levels of innervation in striatal, and lower levels of innervation in the cortical regions. We additionally stained for TPH, a serotonergic marker and found serotonergic neurons in the hindbrain(Figure 10C'). Immunolabeling for TH in the same fusions indicated abundant dopaminergic neurons in the ventral midbrain, and TH⁺ projections into the hindbrain, striatum and cortex can be observed (Figure 10D).

Fusions where both dopaminergic and serotonergic systems are available and can be studied simultaneously could potentially have huge implications in pharmaceutical research, as both systems have strong associations with major depressive disorders and the availability of two monoaminergic systems could allow for a broader investigation of potential drugs.

To summarize, the methodology of recreating the dopaminergic system can be expanded to other, or more groups of different (or the same) brain regions with connective properties, allowing the study of many brain region interactions- and in this specific example allows the study of both serotonergic and dopaminergic pathway-specific features in one tissue.

### Example 10: Morphological reconstruction of neurons

Infecting organoids with an AAV expressing a membrane-bound Arch1-GFP allowed us to morphologically reconstruct neurons from different brain regions and allowed us to observe discrete neuronal morphologies in Cortex, Striatum and ventral Midbrain (Figure 10E). Additionally, it allowed us to observe intricate neuronal morphologies, such as dendritic spines on a dendritic tree of a cortical neuron (Figure 10F).

### Example 11: Neural activity measurements indicate heterogeneous region-specific activity, and functional dopaminergic long-range connections

To investigate features of neuronal activity in the linear fusions, we first investigated if spontaneous neuronal activity could be observed. For this, we grew organoid fusions with an H9 cell line expressing the fluorescent calcium indicator GCAMP6s under a synapsin promoter, which restricts GCAMP expression to neurons. Notably, we always only used this cell line in one of the three regions, which restricts GCAMP expression to cells which received either cortical, striatal or ventral midbrain patterning. On day 120, organoids were recorded for 6.5 min and the recordings were processed using an adapted version of the CaImAn calcium imaging analysis pipeline (Flatiron Institute). We could observe different modes of activity in Cortex, Striatum and Midbrain. Cortical organoids often, but not always, displayed repetitive synchronous network events (Figure 11A, B), whereas neuronal activity in the striatum was usually more disorganized, and the calcium transients lasted longer. Ventral midbrain neurons displayed events of high-frequency synchronous calcium transients (Figure 11A, B, end of the recording), which was unique to recordings from the ventral midbrain.

We additionally looked at fusions with GCAMP in the ventral midbrain and investigated if we could observe calcium transients in other regions in axons projecting from the ventral midbrain. As can be seen in Figures 11C and D, calcium transients could well be observed in axons projecting from the ventral midbrain into the striatum, confirming that neuronal activity of one region can also spread to other regions.

To confirm functional axonal connections, we combined the optogenetic tool Channelrhodopsin2 with extracellular recordings using silicon probes. In brief, we infected organoids with an AAV-RG containing a CAG-hChR2-H134R-tdtomato and incubated them for 2 weeks. We then stimulated the ventral midbrain side of a triple fusion using an optogenetic setup and recorded from striatum and cortex using silicon probes (Figure 11E, F). Notably, we could get direct responses from ventral midbrain stimulations in the striatum (Figure 11G, H, I), indicating the establishment of functional long-range connections from the ventral midbrain.

We could additionally observe a similar phenotype in the cortical part of the fusion.

To confirm not only the establishment of functional long-range connections, but also to test whether these connections were of dopaminergic identity, we performed another optogenetic stimulation experiment in composition with a fluorescent genetic dopaminergic sensor (GRAB-DA4.4). We infected organoids with AAV1 containing Syn-ChrimsonR-tdTomato (as hChR2 is incompatible with GRAB-DA4.4) and incubated them for two weeks. We then recorded the fluorescence intensity of the ventral midbrain before and during optogenetic stimulation and could observe a change in fluorescence in many of the recorded neurons, indicating dopamine release (Figure 11K, L).

To summarize, we could show that we have spontaneously active neurons (GCAMP), that they project into other regions, form functional connections with the target neurons (silicon probes) and that stimulation of the ventral midbrain organoid results in a release of dopamine in target regions (dopamine sensors). This allows us to ask dopamine circuit-specific questions.

### Example 12: Using triple fusions to test GMP grade mDA progenitors for cell therapy

Some of the most promising therapies for the currently uncurable Parkinson's disease is the transplantation of dopaminergic neurons into patient brains. However, for highly efficient cell therapy, several steps still have to be improved and several caveats limit current studies.

Firstly, mostly A9 dopaminergic neurons are affected (although other dopaminergic neurons such as olfactory bulb dopaminergic neurons and A10 dopaminergic neurons can also be affected but are of less vital importance). However, current grafts contain more A10 than A9 dopaminergic neurons, and it is currently unknown how to increase the proportions of A9 dopaminergic neurons in grafts. A system which allows fast screening of conditions, but also allows the interaction of neurons with its targets - an important step in dopaminergic maturation - could be ideal for testing such new conditions, without the need to go into slow, expensive and tedious animal experiments.

Secondly, dopaminergic grafts are currently grafted into the striatum, as grafts in the ventral midbrain did not show the potential to re-innervate the (in the human) long distance into the striatum.

However, recent studies have shown that overexpression of axonal chemoattractants, such as GDNF, could recruit dopaminergic axons into the striatum in mouse experiments (Niamh et al., Cell Stem Cell, 2022). While this system seems to work in mice, dopaminergic axons have to migrate over much longer distances in the human brain, suggesting more sophisticated recruitment strategies might be necessary to recruit dopaminergic axons over longer distances.

We here propose the usage of linearly fused organoids to study mDA grafts in a 3D environment, which would allow high throughput and better accessibility than animal model systems. To investigate this possibility, we injected 40.000 GMP grade RC17 embryonic stem cell derived mDA progenitors in 200nl into the ventral midbrain part of a triple fusion (Figure 12A, B). These cells were modified with an endogenous Cre in the TH locus, and double infected with a Lentivirus containing a floxed GFP, thus labeling dopaminergic neurons with GFP. One month after injection, the graft can be readily observed in the ventral midbrain. Surface confocal microscopy revealed high levels of axonal innervation of the midbrain, but also axon bundles projecting to the striatum, and innervation of striatal and cortical regions (Figure 12C).

We next performed live-imaging of dopaminergic axons and could observe movement along the axon (Figure 12E), which could be indicative of axonal transport, as well as dopaminergic axon-axon interactions (Figure 12F).

We performed 3D tissue clearing and could recreate the dopaminergic grafts (GFP⁺) and FOXA2⁺ cells from the graft, with some of them already differentiating into TH⁺ neurons (Figure 12G).

## Claims

1. An *in vitro* method of producing a fused tissue culture with at least three different tissues comprising
selecting a spatial shape for attaching the at least three different tissues to each other,
placing the at least three different tissues into contact in said spatial shape,
culturing the at least three different tissues under conditions that maintains tissue fusion of the at least three different tissues.

2. The method of claim 1, wherein at least one, preferably at least two, of the at least three different tissues are in contact with only one of the at least three different tissues.

3. The method of claim 1 or 2, wherein the spatial shape is a planar or linear shape or the spatial shape follows the curvature of a carrier on which the at least three different tissues are placed.

4. The method of any one of claims 1 to 3, wherein the at least three different tissues are placed onto a carrier, wherein the carrier has a slope gradually descending to a deepest depression, wherein the at least three different tissues sink to the bottom of the carrier and then are pushed together due to a downslope force caused by an inclination of the slope; preferably wherein the slope being lower inclined closer to the deepest depression than further away from the deepest depression of the carrier, or preferably wherein the slope gradually descending to a deepest depression is a curved slope.

5. The method of any one of claims 1 to 4, comprising placing the at least three different tissues in a recess of a carrier, wherein said recess is elongated and comprises a deepest depression and at least one slope, the at least one slope descending to the deepest depression,
wherein the placement of the at least three different tissues into the recess forces contacts of each the at least three different tissues with at least one other of the at least three different tissues through gravity in the recess; preferably wherein the recess has a length of 1 mm to 40 mm and/or preferably a width of 0.5 mm to 16 mm.

6. The method of any one of claims 1 to 5, wherein the at least three different tissues comprise 1, 2 or 3 different brain or neural or neuronal tissues; preferably wherein at least one of the different tissues is a dopaminergic neuronal tissue or a serotonergic neuronal tissue.

7. An in vitro method of generating a ventral hindbrain tissue culture, comprising inducing neural differentiation in pluripotent cells or multipotent neural stem cells with a neural induction medium comprising at least one TGF-beta inhibitor and a Wnt activator, and allowing the cells during said induction to form a cell aggregate;
culturing the cell aggregate to form a tissue culture.

8. A tissue culture comprising a planar or linear fusion of at least three different neural or neuronal tissues; preferably having a size of 300 µm to 30 mm in its longest dimension.

9. The tissue culture of claim 8, comprising neural cells with a cell body in one of said at least three different neural or neuronal tissues and an axon to another one of said at least three different neural or neuronal tissues, preferably wherein a tissue comprises neuronal cells that project their axons into at least two of the other brain tissues.

10. The tissue culture of claim 8 or 9, wherein the at least three different brain tissues are selected from central nervous system tissue,
preferably of a telencephalic tissue, in particular preferred of tissues of cortical, striatal, lateral ganglionic eminence (LGE), medial ganglionic eminence (MGE) or caudal ganglionic eminence (CGE) identity;
preferably of a diencephalic tissue, especially preferred thalamic, hypothalamic, epithalamic, subthalamic or optic cup tissue;
preferably mesencephalic tissue, especially preferred tectum, tegmentum, ventral tegmental area (VTA), substantia nigra (SN), such as SN pars compacta (SNpc), Nucleus ruber tissue; preferably rhombencephalon tissue, especially preferred cerebellum, medulla, pons, raphe nuclei tissue;
preferably spinal cord tissue; or
peripheral nervous system tissue;
preferably the tissue culture comprises cortical tissue; dorsoventral forebrain tissue, preferably lateral ganglionic eminence (LGE) tissue, medial ganglionic eminence (MGE) tissue or caudal ganglionic eminence (CGE) tissue; olfactory tissue, preferably comprising olfactory bulb neurons, amygdala tissue, cortex tissue or hippocampus tissue; limbic system tissue, preferably thalamus, hippocampus, amygdala, hypothalamus; dopaminergic system tissue, preferably ventral tegmental area (VTA), substantia nigra (SN), such as SN pars compacta (SNpc) tissue, nucleus accumbens, olfactory tubercle, prefrontal cortex, cortical, caudate nucleus, putamen, hypothalamus, brainstem, spinal cord tissue; serotonergic system tissue, preferably rostral serotonergic group tissue, such as cortex, striatum, amygdala, substantia nigra, pons, hippocampus, entorhinal cortex, locus coeruleus or caudal serotonergic group tissue, such as trigeminal motor nucleus, dorsal nucleus of vagus nerve, intermediolateral nucleus, medulla, mesencephalon;
cerebellar pathway tissue, preferably cortical, thalamic, pons, vestibular nuclei tissue; noradrenergic pathway tissue, preferably cerebellum, spinal cord, hippocampus, basal ganglia, cortex tissue.

11. The tissue culture of any one of claims 8 to 10, wherein the at least one of the neural or neuronal tissues is selected from ventral hindbrain, ventral midbrain, striatum, cortex, preferably ventral midbrain tissue in contact with striatum tissue and said striatum tissue being in contact with cortex tissue; and/or tissue culture comprising dopaminergic neurons and/or serotonergic neurons, preferably wherein the dopaminergic neurons connect between ventral midbrain tissue and striatum tissue and/or connect between ventral midbrain tissue and cortex tissue.

12. The tissue culture of any one of claims 8 to 11, wherein a neural cell from one of the at least three different neural tissues migrated to at least one of the other at least three different neural or neuronal tissues; and/or wherein the at least three different neural or neuronal tissues comprise a neural or neuronal tissue of a neural or neuronal tissue type and another neural or neuronal tissue of said neural or neuronal tissue type with its cells being modified by a genetic modification or another modification.

13. A method of investigating neural or neuronal formation, comprising introducing a neural stem cell, neural progenitor cell, neuronal cell, neural cell, glial cell, astrocyte or oligodendrocyte into a tissue culture of any one of claims 8 to 12 and monitoring cell differentiation and/or cell growth.

14. A method of testing or screening a candidate compound for influencing neural cells of a fused tissue culture, comprising contacting cells or a tissue in a method of any one of claims 1 to 7 with the candidate compound or contacting a tissue of any one of claims 8 to 12 with the candidate compound and maintaining said contacted tissue in culture, and observing any change in the neural cell as compared to said tissue without contacting by said candidate compound.

15. A mold for tissue fusion comprising a top surface and a recess in said top surface, wherein said recess is elongated and comprises a deepest depression and at least one slope along the direction of the elongation, the at least one slope descending to the deepest depression, wherein the mold at least at the deepest depression comprises a low- or non-cell-adhesion surface; preferably wherein the low- or non-cell-adhesion surface is of a coating with a low- or non-cell-adhesion material.
